# EUROPÄISCHE PATENTSCHRIFT

(11) **EP 2 630 110 B1**
(45) Veröffentlichungstag und Bekanntmachung des Hinweises auf die Patenterteilung: **25.02.2015**
(21) Anmeldenummer: 11772921.0
(22) Anmeldetag: 12.10.2011
(51) Int. Cl.: C07C 41/42, C07C 41/06, C07C 43/04, C07C 1/20, C07C 11/09

(54) **VERFAHREN ZUR AUFREINIGUNG VON MTBE-HALTIGEN GEMISCHEN SOWIE ZUR HERSTELLUNG VON ISOBUTEN DURCH SPALTUNG VON MTBE-HALTIGEN GEMISCHEN**
METHOD FOR PURIFYING METHYL-TERTIARY BUTYL ETHER (MTBE)-CONTAINING MIXTURES AND FOR PRODUCING ISOBUTENE BY CLEAVAGE OF MTBE-CONTAINING MIXTURES
PROCÉDÉ POUR PURIFIER DES MÉLANGES CONTENANT DU MTBE ET POUR PRODUIRE DE L'ISOBUTÈNE PAR DÉCOMPOSITION DE MÉLANGES CONTENANT DU MTBE

(30) Priorität: 21.10.2010 DE 102010042774
(43) Veröffentlichungstag der Anmeldung: 28.08.2013
(73) Patentinhaber: Evonik Degussa GmbH, 45128 Essen (DE)
(72) Erfinder: WINTERBERG, Markus, 45711 Datteln (DE); RÖTTGER, Dirk, 50672 Köln (DE); RIX, Armin, 45770 Marl (DE); BUKOHL, Reiner, 45770 Marl (DE); BÖING, Christian, 50679 Köln (DE)
(86) Internationale Anmeldenummer: PCT/EP2011/067770
(87) Internationale Veröffentlichungsnummer: WO 2012/052327

(56) Entgegenhaltungen:
- DE-A1-102008 040 511
- US-A- 5 159 122

## Beschreibung

Die vorliegende Erfindung betrifft ein Verfahren zur effizienten Aufreinigung von MTBE-haltigen Gemischen sowie zur Herstellung von Isobuten durch Spaltung von MTBE-haltigen Gemischen.

"Schwersieder" im Sinne der Erfindung sind eine Gruppe Komponenten eines flüssigen Stoffgemisches, deren jeweiliger Siedepunkt im Vergleich zu den Siedepunkten der übrigen Komponenten des Stoffgemisches bei höheren Temperaturen liegt, bzw. deren Dampfdruck niedriger ist als die Dampfdrücke der übrigen Komponenten. Die Begriffe "Hochsieder" und "hoch siedende Komponenten" werden synonym zu dem Begriff "Schwersieder" verwendet.

"Leichtsieder" im Sinne der Erfindung sind eine Gruppe Komponenten eines flüssigen Stoffgemisches, deren jeweiliger Siedepunkt im Vergleich zu den Siedepunkten der übrigen Komponenten des Stoffgemisches bei niedrigeren Temperaturen liegt, bzw. deren Dampfdruck höher ist als die Dampfdrücke der übrigen Komponenten. Der Begriff "leichter siedende Komponente" wird synonym zu dem Begriff "Leichtsieder" verwendet.

Isobuten ist ein wichtiges Zwischenprodukt für die Herstellung einer Vielzahl organischer Verbindungen, wie beispielsweise für die Herstellung von Butylkautschuk, Polyisobutylen, Isobuten-Oligomeren, verzweigten C₅-Aldehyden, C₅-Carbonsäuren, C₅-Alkoholen und C₅-Olefinen. Weiterhin wird es als Alkylierungsmittel, insbesondere zur Synthese von tert.-Butylaromaten, und als Zwischenprodukt für die Erzeugung von Peroxiden eingesetzt. Darüber hinaus kann Isobuten als Vorstufe für Methacrylsäure und deren Ester verwendet werden.

In üblichen technischen Strömen liegt Isobuten zusammen mit gesättigten und ungesättigten C₄-Kohlenwasserstoffen vor. Aus diesen Gemischen kann Isobuten wegen der geringen Siedepunktsdifferenz bzw. des geringen Trennfaktors zwischen Isobuten und 1-Buten durch Destillation nicht wirtschaftlich abgetrennt werden. Daher wird Isobuten aus technischen Kohlenwasserstoffen häufig dadurch gewonnen, dass Isobuten zu einem Derivat umgesetzt wird, das sich leicht vom übrigen Kohlenwasserstoffgemisch abtrennen lässt, und dass das isolierte Derivat zu Isobuten und Derivatisierungsmittel zurückgespalten wird.

Üblicherweise wird Isobuten aus C₄-Schnitten, beispielsweise der C₄-Fraktion eines Steamcrackers, wie folgt abgetrennt: Nach Entfernung des größten Teils der mehrfach ungesättigten Kohlenwasserstoffe, hauptsächlich des Butadiens, durch Extraktion und Destillation oder Selektivhydrierung zu linearen Butenen wird das verbleibende Gemisch (Raffinat I oder selektiv hydriertes Crack-C₄) mit Alkohol oder Wasser umgesetzt. Bei der Verwendung von Methanol entsteht aus Isobuten Methyl-tert.-butylether (MTBE) und bei Einsatz von Wasser tert.-Butanol (TBA). Nach ihrer Abtrennung können diese Derivate in Umkehrung ihrer Bildung zu Isobuten gespalten werden.

Technisches MTBE ist eine gefragte Komponente in Kraftstoffen für Ottomotoren zur Erhöhung der Octanzahl. Dabei werden hinsichtlich der Reinheit keine allzu hohen Anforderungen gestellt. Der Gehalt an Methanol und tert.-Butanol darf jeweils bis zu 1 Massen-% betragen. Weiterhin kann technisches MTBE bis zu 0,5 Massen-% C4- bis C8-Kohlenwasserstoffe und bis zu 500 ppm Wasser enthalten. In höheren Reinheiten wird MTBE auch als Löse- und Extraktionsmittel im Pharmabereich und in der Analytik.

Die Spaltung von MTBE zu Isobuten und Methanol kann in Gegenwart saurer oder basischer Katalysatoren in der Flüssigphase bzw. Gas/Flüssig-Mischphase oder in der reinen Gasphase durchgeführt werden. Ein guter Überblick über die bekannten Verfahren findet sich u. a. in R. Trotta und I. Miracca zeigen in Catalysis Today 34 (1997), S 447 bis 455.

In US 5 567 860 wird ein Verfahren zur Herstellung von hochreinem Isobuten beschrieben. Hier werden isobutenhaltige C₄-Ströme zunächst mit Methanol verethert, so dass je nach Umsatz ein Gemisch aus MTBE, 2-Methoxybutan (MSBE), nicht umgesetzten C₄-Kohlenwasserstoffen, Methanol, Wasser, Dimethylether (DME), C₄-Oligomeren sowie C₃- und C₅-Kohlenwasserstoffen als Verunreinigung des C₄-Stroms erhalten wird. Dieses Produktgemisch wird destillativ in Leichtsieder, enthaltend C₃-, C₄- und C₅-Kohlenwasserstoffe, Methanol und DME sowie Hochsieder, enthaltend C₄-Oligomere, getrennt. In einem Seitenabzug der Kolonne werden MTBE und 2-Methoxybutan (MSBE) erhalten, welche dann der sauer katalysierten Spaltung zugeführt werden.

In DE 10 2006 040431 wird ein Verfahren für die Herstellung von Isobuten durch MTBE-Spaltung beschrieben. Dabei wird Einsatz-MTBE zusammen mit einem zurückgeführten MTBE-Strom in einer Kolonne durch Abtrennung von Hochsiedern aufgereinigt und das erhaltene MTBE gespalten. Der Austrag der Reaktion wird destillativ in Isobuten, mit anteiligen (azeotropen) Mengen an Methanol, und eine Mischung mit den Hauptbestandteilen Methanol und unumgesetztes MTBE, getrennt. Aus der Methanol/MTBE-Mischung wird anschließend das Methanol größtenteils entfernt und der MTBE-haltige Strom in die Kolonne zur Abtrennung von Hochsiedern zurückgeführt. Optional werden aus dem Einsatz MTBE Leichtsieder abgetrennt.

Die Schrift DE 10 2006 040430 liegt ein vergleichbares Verfahren zu Grunde. Kennzeichnend sind Aufreinigung des in der Spaltung eingesetzten MTBE auf unter 1000 Massen-ppm 2-Methoxybutan und Konzentrationen an linearen Butenen im erhaltenen Isobuten unter 1000 Massen-ppm. Die Rückführung von nicht umgesetzten MTBE ist optional.

In DE 10 2008 040511 wird zur Ausschleusung mittelsiedender Komponenten der Austrag aus der MTBE-Spaltung, nach Entfernung von Isobuten und des größten Teils des Methanols, in eine Destillation zur Entfernung dieses mittelsiedenden Komponenten gefahren und dann in die Spaltung zurück gefahren. Die Ausschleusung der Mittelsieder kann optional zusammen mit dem im Einsatz-MTBE enthaltenen Leichtsiedern in der Leichtsiederabtrennung erfolgen.

In DE 10 2009 027404 wird eine Kombination von MTBE-Synthese und MTBE-Spaltung beschrieben, wobei nicht umgesetztes MTBE und Methanol von der MTBE-Spaltung zurück in die MTBE-Synthese gefahren wird. Aus diesem Rückführstropm wird Wasser abgetrennt, um eine Aktivierungsminderung des Synthesekatalysators zu vermeiden.

In DE 10231051 wird ein Verfahren zur Herstellung von hochreinem MTBE durch zweimalige Destillation beschrieben. Das Verfahren zeichnet sich durch eine sehr hohe Reinheit des erzeugtem MTBEs aus (> 99,7 Massen-%) sowie durch größere Nebenproduktströmen, die neben den aus dem MTBE abgetrennten Nebenkomponenten wie C4- und C5-Kohlenwasserstoffen, TBA, Methanol und 2-Methoxybutan auch noch nennenswert MTBE enthalten. Das erhaltene MTBE eignet sich als Edukt für eine MTBE-Spaltung zur Herstellung von hochreinem Isobuten.

Die Bildung von Hochsiedern durch Dimerisierung oder Oligomerisierung des Isobutens zu C₄-Oligomeren, sogenannten C₈- und C₁₂-Komponenten, ist eine der bekannten Nebenreaktionen der MTBE-Spaltung. Bei den unerwünschten C₈-Komponenten handelt es sich hauptsächlich um 2,4,4-Trimethyl-1-penten und 2,4,4-Trimethyl-2-penten. Darüber hinaus setzt sich, besonders an basischen Katalysatoren, ein Teil des bei der Spaltung entstehenden Methanols unter Wasserabspaltung zu DME um.

Die weitere Aufarbeitung der in DE 10 2006 040431 und DE 10 2006 040430 erhaltenen methanolhaltigen Isobutenströme sieht daher eine Abtrennung des Methanols durch Extraktion mit Wasser und eine anschließende Destillation vor, bei der DME und Wasser vom Isobuten abgetrennt werden.

Die MTBE-Spaltung in der Gasphase hat den Vorteil, dass sie in der Regel bei höheren Temperaturen abläuft. Das Gleichgewicht der Reaktion von MTBE zu Isobuten und Methanol liegt damit stärker auf Seiten der Produkte, so dass höhere Umsätze erzielt werden können. Wegen der höheren Spalttemperaturen können aber andere und / oder zusätzliche Nebenreaktionen auftreten.

Wie eingangs beschrieben, ist Isobuten ein wichtiges Zwischenprodukt für die Herstellung einer Vielzahl organischer Verbindungen. Die effiziente Herstellung dieser Produkte ist ein Kernbereich der aktuellen industriellen Forschung und stellt dabei gleichzeitig höchste Anforderungen an die Produktreinheit. Tabelle 1 zeigt eine typische Spezifikation für markgängiges Isobuten. Zu beachten ist neben der geforderten hohen Isobutenreinheit (> 99,9 Massen-%) vor allem die scharfe Spezifikation für Oxygenates (max. 10 Massen-ppm). Für Spezialanwendungen kann im Einzelfall sogar eine noch höhere Reinheit gefordert werden.

**Tabelle 1: Typische Zusammensetzung von marktgängigem Isobuten**

| Massenanteile [kg/kg] | |
|---|---|
| C3-Kohlenwasserstoffe | <0,000100 |
| Butane | <0,001000 |
| Isobuten | >0,999000 |
| 1-Buten / 2-Butene | <0,001000 |
| Methanol | <0,000030 |
| C5-Kohlenwasserstoffe | <0,000500 |
| Wasser | <0,000050 |
| Oxygenate | <0,000010 |

| | |
|---|---|
| Oxygenate: Beispielsweise DME, Aceton | |

C4-Schnitte, die für die MTBE-Synthese verwendet werden, enthalten in der Regel aber neben den C4-Kohlenwasserstoffen auch sauerstoffhaltige Verbindungen. Die Art und Menge der sauerstoffhaltigen Verbindungen hängen von der Art und dem Ursprung der eingesetzten Rohstoffströme und den gewählten technischen Bedingungen in den Crackern ab. Auch die weitere Aufarbeitung des C4-Schnitts beeinflusst den Gehalt an diesen Verunreinigungen. Bei den sauerstoffhaltigen Verbindungen handelt es sich beispielsweise um Acetaldehyd und Aceton. Aceton gelangt bei der MTBE-Synthese herstellungsbedingt zumindest anteilig in das MTBE-Produkt. So enthält marktübliches MTBE neben 2-Methoxybutan, C3- bis C₄-Kohlenwasserstoffe, Methanol, Wasser und DME auch Aceton. Wird das MTBE-Produkt zu Herstellung von Isobuten eingesetzt und wird Aceton vor der MTBE-Spaltung nicht abgetrennt, gelangt es in den Reaktionsteil. Hier kann es u.U. zu einer Deaktivierung des Katalysators, z.B. durch Verkoken, beitragen. Wird Aceton nicht umgesetzt, kann es sich im Prozess anreichern und gelangt schließlich ins Isobutenprodukt, was ebenfalls nicht erwünscht ist, da sauerstoffhaltige Verbindungen die Folgeprozesse, in denen das hochreine Isobuten weiterverwendet wird, stören können. Mögliche Folgeprozesse sind z.B. die Herstellung von Polyisobuten (PIB), Butylkautschuk oder auch Methylmethacrylat (MMA).

Keine der erwähnten und in der Literatur beschriebenen MTBE-Aufreinigungs- und MTBE-Spaltungsverfahren sieht die explizite Abtrennung von Aceton vor. Die meisten Verfahren sehen einen Teilumsatz in der MTBE-Spaltung vor, nicht umgesetztes MTBE und Methanol werden in einer nach der Reaktion angeordneten Destillation vom Isobuten abgetrennt und entweder zurück in eine MTBE-Synthese oder zurück in die MTBE-Spaltung gefahren. Bei Abtrennung von Aceton über das Sumpfprodukt dieser Destillation kommt es aber durch die Rückführung des nicht umgesetzten MTBE, entweder in die MTBE-Synthese oder in die MTBE-Spaltung, zu einer Anreicherung des Acetons im Prozess. Passiert Aceton den Reaktor dabei ohne weitere Reaktion, steigt seine Konzentrationen bis zu einer Grenzkonzentration an, bei der es schließlich ins Kopfprodukt der nach dem Reaktor angeordneten Destillation und so letztendlich doch in die Isobutenfraktion gelangt. Durch die Kreislauffahrweise kommt es damit ohne Ausschleusung von Aceton aus dem Prozess im Kreislauf zu einer Anreicherung auf unerwünscht hohe Konzentrationen, bis schließlich Aceton in die Isobutenfraktion gelangt. Die nach dieser Destillation üblicherweise angeordneten Aufarbeitungsschritte - eine Extraktion mit Wasser zur Entfernung von Methanol und eine Destillation zur Entfernung von Dimethylether von Isobuten - führen nicht zu einer Abtrennung von Aceton aus dem Isobutenprodukt. Damit würde dieses bei fehlender Ausschleusung von Aceton aus dem Verfahren verunreinigt und nicht mehr der üblichen Oxygenates-Spezifikation genügen, siehe Tabelle 1. Die dort geforderte Summenspezifikation von < 10 Massen-ppm ist vor allem deshalb sehr anspruchsvoll, da neben Aceton auch weitere Sauerstoffverbindungen, z.B. Dimethylether (DME), herstellungsbedingt im Isobutenprodukt vorhanden sind und ebenfalls abgetrennt werden müssen. Es ist zwar auch noch eine Reinigung des Isobutens, z.B. durch Destillation oder durch Adsorptiion, wie z.B. in EP 1562883 und US2004 0102656 vorgeschlagen, denkbar. Diese Reinigung stellt jedoch einen zusätzlichen Aufarbeitungsschritt dar und würde durch die den damit verbundene höheren apparativen Aufwand sowie die hohen Energieaufwand für die Destillation oder für die Regenerierung des Adsorbenz die Effektivität des Verfahrens unverhältnismäßig mindern.

Bei den aus der Literatur bekannten Verfahren, z.B. DE 10 2009 027404 wird zumindest optional der MTBE-haltige Einsatzstoff (I) durch Destillation von Leichtsiedern befreit. Bei den Leichtsiedern handelt es sich überwiegend um C4- oder C5-Kohlenwasserstoffe. Die Destillationskolonne wird dabei so betrieben, dass die Leichtsieder als Kopfprodukt abgetrennt werden und das Sumpfprodukt die Leichtsieder (C4-Kohlenwasserstoffe, C5-Kohlenwasserstoffe und ggf. auch Sauerstoffverbindungen wie Dimethoxymethan) nur bis zu solchen Grenzkonzentrationen enthält, die die entsprechende Spezifikation im Isobutenprodukt (siehe Tabelle 1) nicht gefährden.

**Tabelle 2: Reinstoffsiedepunkte im Spaltungsverfahren typischerweise vorkommenden Komponenten bei 0,1 und 0,5 MPa_{(abs)}**

| *Reinstoff* | *Siedetemp.[°C] bei 0,1 MPa(abs)* | *Siedetemp.[°C] bei 0,5 MPa(abs)* |
|---|---|---|
| DME | -24,8 | 19,2 |
| Isobuten | -6,9 | 42,7 |
| 1-Buten | -6,3 | 43,4 |
| n-Butan | -0,5 | 50,3 |
| trans-2-Buten | 0,9 | 51,4 |
| cis-2-Buten | 3,7 | 54,1 |
| Isopentan | 27,8 | 83,8 |
| 1-Penten | 30,0 | 85,5 |
| Isopren | 34,1 | 90,4 |
| n-Pentan | 36,1 | 92,6 |
| Dimethoxymethan | 41,9 | 95,9 |
| Methanol | 64,7 | 111,5 |
| Aceton | 56,3 | 111,7 |
| MTBE | 55,2 | 113,8 |
| 2-Methoxybutan | 59,0 | 120,8 |
| TBA | 82,4 | 131,3 |
| Diisobuten | 101,4 | 171,2 |

In Tabelle 2 sind für verschiedene Komponenten, die im erfindungsgemäßen Verfahren typischerweise entweder im MTBE-haltigen Einsatzstoff enthalten sind oder aber im Reaktionsteil gebildet werden, die Normalsiedepunkte aufgeführt. Da viele Destillationen im erfindungsgemäßen Verfahren vorzugsweise unter erhöhtem Duck durchgeführt werden, sind zusätzlich die Siedepunkte bei 0,5 MPa_{(abs)} mit in der Tabelle aufgeführt. Wie zu sehen, siedet Aceton sehr nahe bei MTBE, bei erhöhtem Druck siedet Aceton zwischen MTBE und Methanol. Darüber hinaus bildet Aceton sowohl mit Methanol als auch mit MTBE Azeotrope. Daher gelangt Aceton bei üblicher Fahrweise der Destillation zur Leichtsiederabtrennung nicht ins Destillat und wird daher nicht abgetrennt.

Prinzipiell ist zwar eine gleichzeitige Abtrennung von Aceton in dieser Destillation denkbar. Üblichweise wird die Leichtsiederabtrennung so betrieben, dass das Sumpfprodukt weitestgehend frei von C4- und C5-Kohlenwasserstoffen ist und dass das Kopfprodukt möglichst wenig MTBE und Methanol enthält, da das Kopfprodukt aus dem Verfahren ausgeschleust wird (MTBE-Konzentration < 25 Massen-%). Nun kann Aceton nur ins Kopfprodukt ausgeschleust werden, in dem die Destillation mit deutlich höheren Destillatmenge (MTBE-Konzentration > 65 Massen-%) und ggf. auch mit gleichzeitigem erhöhtem Rücklauf betrieben wird. Daher ist eine solche Abtrennung durch einfache Destillation des Acetons gemeinsam mit anderen leichtersiedenden Komponenten mit einem unverhältnismäßig hohen Energieaufwand und hohen MTBE- und Methanolverlusten verbunden. Der Destillatstrom ist aufgrund des hohen Anteils and C4- und C5-Kohlenwasserstoffen, ohne erneute Aufarbeitung, auch nicht als Kraftstoffadditiv geeignet.

Aufgabe der vorliegenden Erfindung war deshalb die Bereitstellung eines Verfahrens zur Herstellung von hochreinem MTBE aus technischem MTBE, bei dem das technische MTBE auf effektive und wirtschaftliche Weise von Aceton befreit wird und damit durch das Verfahren ein MTBE gewonnen wird, das u.a. zur Herstellung von hochreinem Isobuten geeignet ist.

Gelöst wurde die Aufgabe dadurch, dass der MTBE-haltige Einsatzstoff einer Destillation unterworfen wird, bei der das Aceton überwiegend in einem Seitenstrom entfernt wird. Neben Aceton enthält der Seitenstrom vorwiegend MTBE und Methanol. Der Kopfstrom der Destillation enthält überwiegend C4- und C5-Kohlenenwasserstoffe, und der Sumpfstrom enthält überwiegend MTBE.

Gegenstand der Erfindung ist deshalb ein Verfahren zur Aufreinigung von technischem MTBE, das folgende Schritte umfasst:
a) Bereitstellen von technischem MTBE (I), enthaltend mindestens MTBE, Methanol, C4-Kohlenwasserstoffe, C5-Kohlenwasserstoffe und Aceton; und
b) destillatives Trennen des technischen MTBE (I) in ein C4- und C5- Kohlenwasserstoffe enthaltendes Kopfprodukt (II), einen Aceton, Methanol und MTBE enthaltenden Seitenstrom (III), und ein MTBE enthaltendes Sumpfprodukt (IV).

Das erfindungsgemäße Verfahren weist im Vergleich zum nächsten Stand der Technik, beschrieben in DE 10 2006 040431, DE 10 2006 040430 und DE 10238370 den Vorteil auf, dass Aceton gezielt und effektiv ausgeschleust wird.

Vorzugsweise wird das destillative Trennen in Verfahrensschritt b) so durchgeführt, dass das Sumpfprodukt (IV) weniger als 50 Massen-% des im technischen MTBE (I) enthaltenen Acetons enthält.

Das destillative Trennen in Verfahrensschritt b) wird dabei typischerweise in einer Destillationskolonne durchgeführt und der Seitenstrom (III) flüssig entnommen.

Aus dem in Schritt b) erhaltenen Sumpfprodukt (IV) können optional auch Schwersieder, vor allem C8-Kohlenwasserstoffe, in einem Verfahrensschritt c) entfernt werden. Daher wird in einer bevorzugten Ausführungsform nach Verfahrensschrittes b) das Sumpfprodukt (IV) der ersten Destillation in einer weiteren Destillation weiter aufgereinigt. In dieser Destillation können höher als MTBE siedende Komponenten, also Schwersieder, vor allen C8-Kohlenwasserstoffe, als Sumpfprodukt (V) entfernt werden. Eine weitere Aufgabe dieser Kolonne kann die teilweise oder vollständige Abtrennung von 2-Methoxybutan sein; denn 2-Methoxybutan kann im Reaktionsteil zu linearen Butenen und Methanol gespalten werden; lineare Butene können bei zu hoher Konzentration gegebenenfalls die Isobuten-Spezifikation gefährden. Das MTBE-haltige Kopfprodukt (VI) der Destillation kann ggf. aufgrund seiner Reinheit als Löse- und Extraktionsmittel im Pharmabereich verwendet werden.

Eine bevorzugte Ausführungsform des erfindungsgemäßen Verfahrens sieht somit vor, dass das Sumpfprodukt (IV) aus Verfahrensschritt b) in einem Verfahrensschritt c) in einer weiteren Destillation in ein MTBE-haltiges Kopfprodukt (VI) und ein Sumpfprodukt (V), das höher als MTBE siedende Komponenten aufweist, aufgetrennt wird.

Das destillative Trennen in Verfahrensschritt c) kann vorzugsweise so durchgeführt wird, dass das MTBE-haltige Kopfprodukt (VI) eine Konzentration von weniger als 2500 Massen-ppm an 2-Methoxybutan aufweist.

Die destillativen Trennungen werden vorzugsweise so durchgeführt, dass das MTBE enthaltende Sumpfprodukt (IV) oder das MTBE-haltige Kopfprodukt (VI) weniger als 50 Massen-ppm an Aceton enthält.

Der Strom (IV) bzw., nach Schwersiederabtrennung, Strom (VI) ist besonders geeignet als Einsatzstoff zur Herstellung von hochreinem Isobuten durch katalytische Spaltung.

Eine bevorzugte Ausführungsform des erfindungsgemäßen Verfahrens sieht somit vor, dass das MTBE enthaltende Sumpfprodukt (IV) oder das MTBE-haltige Kopfprodukt (VI) in einem Verfahrensschritt d) katalytisch gepalten wird unter Erhalt eines Spaltproduktes (VII), welches zumindest MTBE, Isobuten und Methanol enthält. Die Spaltung wird bevorzugt an einem festen Katalysator in der Gasphase in einem Temperaturbereich von 150 bis 500 °C durchgeführt.

Das in Verfahrensschritt d) gewonnene Spaltprodukt (VII) in einem Verfahrensschritt e) in einer weiteren Destillation in ein Isobuten enthaltendes Kopfprodukt (IX) und ein MTBE und Methanol enthaltendes Sumpfprodukt (VIII) aufgetrennt wird.

Gegenstand der Erfindung ist somit auch die Herstellung von hochreinem Isobuten, gekennzeichnet durch Schritte:
d) Spalten des in Strom (IV) bzw. in Strom (V) enthaltenen MTBE an einem heterogenen Katalysator unter Erhalt eines Stromes (VII), welcher mindestens MTBE, Methanol und Isobuten enthält, und
e) destillatives Trennen des Stroms (VII) in ein Kopfprodukt (IX), mindestens enthaltend Isobuten, und ein Sumpfprodukt (VIII), mindestens enthaltend MTBE und Methanol.

Das in Verfahrensschritt e) gewonnene und Isobuten enthaltende Kopfprodukt (IX) enthält dabei vorzugsweise weniger als 10 Massen-ppm an Aceton.

Das in Strom (VIII) enthaltene MTBE wird vorzugsweise in den Reaktionsteil der MTBE-Spaltung zurückgeführt. Dazu wird vorzugsweise in einer weiteren Destillation das Methanol als Sumpfprodukt entfernt und ein MTBE und Methanol enthaltener Strom zur Spaltung zurück gefahren. Besonders bevorzugt erfolgt die Rückführung in Schritt b) des erfindungsgemäßen Verfahrens. Alternativ kann die Rückführung auch in eine MTBE-Synthese erfolgen, wobei eine Methanolabtrennung nicht erforderlich ist, aber vorteilhaft sein kann. Bevorzugt erfolgt die Rückführung in die MTBE-Synthese, aus der auch der MTBE-haltige Strom für Verfahrensschritt a) stammt.

In einer bevorzugten Ausführungsform des erfindungsgemäßen Verfahrens kann aus dem in Verfahrensschritt e) gewonnenen und Isobuten enthaltenden Kopfprodukt (IX) in einem weiteren Verfahrensschritt f) Methanol durch Extraktion und/oder Dimethylether durch Destillation entfernt werden.

Besonders bevorzugt wird dabei das Methanol aus dem Isobuten enthaltenden Kopfprodukt (IX) durch Extraktion mittels eines Extraktionsmittels (XIII) und Abführen eines Methanol enthaltenden Extraktionsstroms (XI) und Abführen eines Isobuten angereicherten Stroms (X) entfernt.

In einer bevorzugten Ausführungsform des erfindungsgemäßen Verfahrens kann der Methanol enthaltende Extraktionsstrom (XI) in einem weiteren Verfahrensschritt in einer Destillation in ein Methanol enthaltendes Kopfprodukt (XII) und ein das Extraktionsmittel (XIII) enthaltende Sumpfprodukt (XIII) aufgetrennt werden.

Weiterhin kann das in Verfahrensschritt e) gewonnene und MTBE und Methanol enthaltende Sumpfprodukt (VIII) ganz oder teilweise, ggf. auch nach weiterer Aufarbeitung, in Verfahrensschritt b) zurückgeführt werden. Dabei kann das Sumpfprodukt (VIII) in Verfahrensschritt a) anstelle des technischen MTBE (I) oder zusätzlich zum technischen MTBE (I) bereitgestellt und dann im Verfahrensschritt b) destillativ getrennt werden.

In einer bevorzugten Ausführungsform des erfindungsgemäßen Verfahrens kann der MTBE und Methanol enthaltende Sumpfstrom (VIII) in einem weiteren Verfahrensschritt in einer Destillation in ein Methanol enthaltendes Sumpfprodukt (XVIII) ein MTBE enthaltendes Kopfprodukt (XVII) aufgetrennt werden, wobei das Kopfprodukt (XVII) ganz oder teilweise in Verfahrensschritt b) zurückgeführt wird.

In einer alternativen Ausführungsform kann in einem weiteren Verfahrensschritt der MTBE und Methanol enthaltende Sumpfstrom (VIII) sowie mindestens ein weiterer methanolhaltiger Strom (XXI) und ein isobutenhaltiger Strom (XX) einer MTBE-Synthese zugeführt und ein MTBE-haltiges Produkt (XXII) ganz oder teilweise in Verfahrensschritt b) zurückgeführt werden.

Das in Verfahrensschritt e) gewonnene Isobuten (IX), das erfindungsgemäß nahezu frei an Aceton ist und das vorzugsweise zu größer 95 Massen-% aus Isobuten besteht, kann nun direkt als Verkaufsprodukt eingesetzt werden oder aber, wie erwähnt, weiter aufgereinigt werden. Vorzugsweise wird das Isobuten (IX) in einem Verfahrenschritt f) weiter aufgearbeitet. Dabei kann das in Strom (IX) enthaltene Methanol nach bekannten Verfahren, beispielsweise durch Extraktion, abgetrennt werden. Die Extraktion von Methanol aus Strom (IX) kann beispielsweise mit Wasser oder einer wässrigen Lösung als Extraktionsmittel z. B. in einer Extraktionskolonne durchgeführt werden.

Der feuchte Isobutenstrom (X) aus der Extraktion kann in einer weiteren Destillation von Dimethylether und Wasser getrennt und zu trockenem Isobuten aufgearbeitet werden. Das trockene Isobuten wird dabei als Sumpfprodukt (XVI) erhalten. Im Kondensationssystem am Kopf der Kolonne wird nach Phasentrennung Wasser (XIV) flüssig und Dimethylether (XV) gasförmig abgezogen.

Nachfolgend wird die Erfindung beispielhaft beschrieben, ohne dass die Erfindung, deren Schutzbereich sich aus den Ansprüchen und der Beschreibung ergibt, darauf beschränkt sein soll. Auch die Ansprüche selbst gehören zum Offenbarungsgehalt der vorliegenden Erfindung. Sind nachfolgend Bereiche, allgemeine Formeln oder Verbindungsklassen angegeben, so soll die Offenbarung nicht nur die entsprechenden Bereiche oder Gruppen von Verbindungen erfassen, die explizit erwähnt sind, sondern auch alle Teilbereiche und Teilgruppen von Verbindungen, die durch Weglassen von einzelnen Werten (Bereichen) oder Verbindungen erhalten werden können, ohne dass diese aus Gründen der besseren Übersichtlichkeit explizit genannt worden sind.

### Verfahrenschritt a): Bereitstellung des MTBE-haltigen Einsatzstoffes

Die vorliegende Erfindung betrifft ein Verfahren zur effizienten Aufreinigung von MTBE-haltigen Gemischen sowie zur Herstellung von Isobuten durch Spaltung von MTBE-haltigen Gemischen. Es kann MTBE unterschiedlicher Qualität eingesetzt werden. Insbesondere können technisches MTBE verschiedener Qualitäten oder Gemische aus technischem MTBE und Methanol eingesetzt werden. Technisches MTBE (Kraftstoffqualität) ist daher der bevorzugte Einsatzstoff. Tabelle 3 zeigt beispielsweise die typische Zusammensetzung eines technischen MTBE.

**Tabelle 3: Typische Zusammensetzung von technischem MTBE (Kraftstoffqualität)**

| *Komponente* | *Anteil* | *Einheit* |
|---|---|---|
| C4-KWSt | 100 - 1200 | Massen-ppm |
| C5-KWSt | 500 - 2000 | Massen-ppm |
| MTBE | 97 - 99,0 | Massen-% |
| 2-Methoxybutan (MSBE) | 1000 - 3500 | Massen-ppm |
| Methanol | 0,3 - 1,0 | Massen-% |
| tert.-Butanol | 1000-5000 | Massen-ppm |
| Wasser | 5-500 | Massen-ppm |
| C8-KWSt | 1000 - 4000 | Massen-ppm |
| Aceton | 10-500 | Massen-ppm |

Technisches MTBE kann nach bekannten Verfahren durch Umsetzung von C4-Kohlenwasserstoffgemischen, aus denen die mehrfach ungesättigten Kohlenwasserstoffe weitgehend entfernt worden sind, beispielsweise Raffinat I oder selektiv hydriertes Crack-C4, mit Methanol hergestellt werden (MTBE-Synthese). Prinzipiell können hierzu alle bekannten Verfahren zur MTBE-Synthese eingesetzt werden, beispielsweise kann die MTBE-Synthese analog zur Beschreibung in DE 101 02 082 erfolgen.

Die MTBE-Synthese wird vorzugsweise in mindestens zwei, besonders bevorzugt in drei Festbettreaktoren durchgeführt. Als Reaktoren, in denen das Methanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Aus dem jeweils letzten der Festbettreaktoren wird eine MTBE-haltige Reaktionsmischung abgezogen. Die destillative Abtrennung der MTBE-haltigen Fraktion aus der Reaktionsmischung kann im einfachsten Fall in einer einzigen Kolonne erfolgen. Diese Kolonne kann auch als Reaktivdestillation ausgeführt werden, um in der Reaktionsmischung noch vorhandenes Isobuten weiter abzureagieren. Als Sumpfprodukt wird dabei technisches MTBE erhalten, das als Einsatzstoff (I) für die MTBE-Spaltung dienen kann. Das Destillat besteht hauptsächlich aus nicht umgesetzten C4-Kohlenwasserstoffen, die aufgrund der Azeotropbildung mit Methanol auch Methanol enthalten. Das Sumpfprodukt enthält neben MTBE herstellungsbedingt noch andere Komponenten wie z.B. 2-Methoxybutan, C4,-, C5-, C8-Kohlenwasserstoffe und Wasser, die entweder als Nebenprodukt in der MTBE-Synthese gebildet werden oder bereits im Einsatzstoff enthalten sind und nicht abgetrennt werden. Abhängig vom gewählten Verhältnis Methanol / Isobuten und dem erreichten Umsatz kann auch noch Methanol enthalten sein. Im erfindungsgemäßen Verfahren wird die MTBE-Synthese und die Destillation bevorzugt so betrieben, dass der Methanolgehalt möglichst gering ist.

Die Einsatzströme für die MTBE-Synthese enthalten in der Regel neben aber neben den C4-Kohlenwasserstoffen auch sauerstoffhaltige Verbindungen. Die Art und Menge der sauerstoffhaltigen Verbindungen hängen von der Art und dem Ursprung der eingesetzten Rohstoffströme und den gewählten technischen Bedingungen in den Crackern ab. Auch die weitere Aufarbeitung des C4-Schnitts beeinflusst den Gehalt an diesen Verunreinigungen. Bei den sauerstoffhaltigen Verbindungen handelt es sich inbsondere um Aceton. Aceton wird in der MTBE-Synthese und in der folgenden Destillation nicht vom MTBE abgetrennt und gelangt herstellungsbedingt zumindest anteilig in das MTBE-Produkt. Wie Tabelle 3 zu entnehmen ist, kann technisches MTBE, je nach Herstellungsprozess und verwendeten Rohstoffstrom, bis zu 500 ppm Aceton enthalten. Aufgabe der Erfindung ist es, das Aceton möglichst vollständig und effektiv aus dem MTBE zu entfernen.

### Verfahrensschritt b): Leichtsieder- und Aceton-Abtrennung

Wie bereits oben ausgeführt, wird bei aus der Literatur bekannten Verfahren zumindest optional der MTBE-haltige Einsatzstoff (I) durch Destillation von Leichtsiedern befreit. Bei den Leichtsiedern handelt es sich überwiegend um C4- oder C5-Kohlenwasserstoffe. Die Destillationskolonne wird dabei so betrieben, dass die Leichtsieder als Kopfprodukt abgetrennt werden und das Sumpfprodukt die Leichtsieder nur bis zu solchen Grenzkonzentrationen enthält, die die entsprechende Spezifikation im Isobutenprodukt (siehe Tabelle 1) nicht gefährden. Bei dieser Fahrweise wird Aceton nicht abgetrennt. Es wurde zwar gefunden, dass durch veränderte Fahrweise bzw. Auslegung der Destillation Aceton prinzipiell auch durch deutliche Erhöhung der Destillatmenge und ggf. auch des Rücklaufverhältnisses über Kopf anteilig abgetrennt werden kann. Doch ist eine solche Abtrennung durch einfache Destillation des Acetons gemeinsam mit anderen leichtersiedenden Komponenten als Kopfprodukt mit einem unverhältnismäßig hohen Energieaufwand und zu hohen MTBE- und Methanolverlusten verbunden und daher nicht bevorzugt.

Erfindungsgemäß erfolgt die Actonabtrennung in einer Destillationskolonne K1, in der das MTBE-haltige Gemisch (I) in ein Kopfprodukt (II), das vorwiegend die C4-und C5-Kohlenwasserstoffe enthält, einen Seitenstrom (III), der Aceton, Methanol und MTBE enthält, und einen Sumpfstrom (IV), der MTBE enthält, aufgetrennt wird.

Vorzugsweise wird diese Stofftrennung in einer Destillationskolonne mit Seitenabzug durchgeführt, die 20 bis 80 theoretische Trennstufen, bevorzugt 25 bis 60 und besonders bevorzugt 35 bis 55 theoretische Trennstufen aufweist. Die Aufgabe des Zulaufstromes erfolgt unterhalb des Seitenabzuges, bevorzugt mindestens 5 Stufen unterhalb, besonders bevorzugt mindestens 10 Stufen unterhalb. Der Abzug des Seitenstromes erfolgt bevorzugt zwischen Stufe 5 und 25, gezählt von oben, besonders bevorzugt zwischen Stufe 10 und 20. Vorzugsweise wird die Kolonne, in Abhängigkeit von der realisierten Stufenzahl, der Zusammensetzung des eingesetzten MTBE und der erforderlichen Reinheit des Sumpf- und Kopfproduktes und des Seitenstroms mit einem Rücklaufverhältnis zwischen 100 und 800, insbesondere zwischen 150 und 750 betrieben. Das Rücklaufverhältnis ist hierbei definiert als Massenstrom des Rücklaufs geteilt durch den Massenstrom des Destillats. Das Verhältnis von Massenstrom des Seitenabzugs (III) zu dem Massenstrom des Zulaufs (I) zur Kolonne beträgt vorzugsweise 0,01 bis 0,2, besonders bevorzugt 0,05 bis 0,01. Der Abzug des Seitenstroms (III) erfolgt bevorzugt flüssig. Die Kolonne wird vorzugsweise mit einem Betriebsdruck von 0,2 bis 0,6 MPa(abs), bevorzugt von 0,3 bis 0,4 MPa(abs) betrieben. Zur Beheizung der Kolonne kann z. B. Dampf eingesetzt werden. Die Kondensation kann, je nach gewähltem Betriebsdruck, gegen Kühlsole, Kühlwasser oder Luft erfolgen. Der Kopfbrüden der Kolonne kann vollständig oder nur teilweise kondensiert werden, so dass das Kopfprodukt (II) entweder flüssig oder dampfförmig abgezogen werden kann. Das Kopfprodukt (II) kann thermisch verwertet werden oder als Einsatzstoff anderer Prozesse, beispielsweise in einer Synthesegasanlage, genutzt werden.

Vorzugsweise wird, durch Einstellung der Destillationsbedingungen, größer 50 Massen-% des zugeführten Acetons durch den Seitenstrom (II) abgeführt werden, besonders bevorzugt größer 75 Massen-%. Das Sumpfprodukt (IV) enthält vorzugsweise weniger als Massen-50 % des zugeführten Acetons, besonders bevorzugt weniger als Massen-80 %. Die Konzentration an Aceton beträgt im Sumpfprodukt (IV) bevorzugt weniger als 50 ppm, besonders bevorzugt weniger als 30 ppm.

Es sei darauf hingewiesen, dass die Acetonabtrennung auch in einer Trennwandkolonne erfolgen kann. Trennwandkolonnen sind Destillationskolonnen, die in Teilbereichen eine Längsunterteilung aufweisen. Diese als fest eingeschweißte oder lose eingesteckte Wand ausgeführte Längsunterteilung verhindert in dem betreffenden Teilbereich der Kolonne eine Quervermischung von Flüssigkeits- und Brüdenströmen. Bei einer Ausfügrung der Kolonne K1 als Trennwandkolonne wird der Seitenstrom (III) im Bereich der Trennwand abgezogen. Dies hat den Vorteil, dass eine größere Anreichung an Aceton im Seitenabzug (III) erfolgt und der Seitenabzug (III) weniger C4- und C5-Kohlenwasserstoffe enthält.

### Verfahrensschritt c): Hochsieder-Abtrennung

In einer bevorzugten Ausführungsform wird nach der ersten Destillation zur Abtrennung von Aceton und Leichtsiedern in Verfahrensschritt b) in einem Verfahrensschritt c) der MTBE-Strom (IV) in einer weiteren Destillation weiter aufgereinigt. In diesem Schritt werden Schwersieder, vor allen C8-Kohlenwasserstoffe wie Diisobuten, als Sumpfprodukt entfernt. Eine weitere Aufgabe dieser Kolonne kann die teilweise oder vollständige Abtrennung von 2-Methoxybutan sein, denn 2-Methoxybutan kann im MTBE-Spaltungsreaktor zu linearen Butenen und Methanol gespalten werden. Lineare Butene können bei zu hoher Konzentration gegebenenfalls die Isobuten-Spezifikation gefährden.

Die destillative Trennung des von Aceton und Leichtsiedern befreiten MTBE-Stroms (IV) in einen MTBE aufweisenden Kopfstrom (VI) und einen Sumpfstrom (V), der höher als MTBE siedende Verbindungen aufweist, gemäß Verfahrensschritt c) erfolgt vorzugsweise in zumindest einer Kolonne, bevorzugt in genau einer Destillationskolonne K2.

Wenn in der Kolonne K2 insbesondere nur C8-Kohlenwasserstoffe abgetrennt werden sollen, kann es vorteilhaft sein, wenn die Kolonne 15 bis 60 theoretische Trennstufen, vorzugsweise 20 bis 55 und bevorzugt 30 bis 45 theoretische Trennstufen aufweist. Das Rücklaufverhältnis, im Rahmen der vorliegenden Erfindung definiert als der Massenstrom des Rücklaufs geteilt durch den Massenstrom des Destillats, wird, in Abhängigkeit von der realisierten Stufenzahl, der Zusammensetzung des eingesetzten MTBE und der erforderlichen Reinheit, vorzugsweise auf einen Wert von 0,5 bis 7, bevorzugt von 1 bis 4 eingestellt.

Sollen in der Kolonne K2 C8-Kohlenwasserstoffe und zusätzlich 2-Methoxybutan abgetrennt werden, weist die eingesetzte Destillationskolonne vorzugsweise von 50 bis 140 theoretische Trennstufen, bevorzugt von 60 bis 120 und ganz besonders bevorzugt von 80 bis 110 auf. Das Rücklaufverhältnis beträgt, in Abhängigkeit von der realisierten Stufenzahl, der Zusammensetzung des eingesetzten MTBE und der erforderlichen Reinheit, vorzugsweise von 1 bis 20, bevorzugt von 2,5 bis 10. Selbst wenn die Abtrennung von 2-Methoxybutan nicht notwendig sein sollte, muss die Auslegung der Kolonne mit höherer Bodenzahl kein Nachteil sein, da ein Teil des höheren Kapitaleinsatzes für die größere Kolonne durch Energieeinsparung (Verringerung des Rücklaufverhältnisses) kompensiert werden kann. Gleichzeit erhält man hierdurch eine höhere operative Flexibilität.

Der Betriebsdruck der Kolonne K2 kann vorzugsweise von 0,1 bis 2,0 MPa(abs) betragen. Wenn eine Spaltung der am Kolonnenkopf erhaltenen MTBE-Fraktion (VI) im MTBE-Spaltreaktor in Verfahrenssschritt d) in der Gasphase bei erhöhtem Druck erfolgt, kann es vorteilhaft sein, die Destillation bei höherem Druck durchzuführen, wobei in diesem Fall der Kopfkondensator vorzugsweise als Partialkondensator betrieben wird und das Kopfprodukt (VI) dampfförmig abgezogen wird. Das dampfförmig abgezogene Kopfprodukt kann dann direkt oder nach weiterer Vorwärmung dem Reaktor zugeführt werden. Der Druckunterschied zwischen Destillation und Reaktor beträgt hierbei bevorzugt mindestens 0,05 MPa_{(abs)}. Beträgt der Reaktionsdruck im Spaltreaktor beispielsweise 0,7 MPa_{(abs)}, so sollte der Destillationsdruck vorzugsweise mindestens 0,75 MPa_{(abs)} betragen. Bei Betriebsdrucken von größer 0,95 MPa_{(abs)} kann mit der Kondensationswärme (Niederdruck-) Dampf erzeugt werden, mit dem andere Kolonnen des Verfahrens beheizt werden können. Zur Beheizung der Kolonne kann, je nach gewähltem Betriebsdruck, Dampf oder Wärmeträgeröl eingesetzt werden.

Das Kopfprodukt (VI) der Kolonne K2 enthält bevorzug weniger als 2500 ppm 2-Methoxybutan, besonders bevorzug weniger als 2000 ppm. Strom (VI) kann aufgrund seiner Reinheit als Löse- und Extraktionsmittel im Pharmabereich und in der Analytik verwendet werden. Bevorzugt dient dieser Strom aber als Zulaufstrom zu einer MTBE-Spaltung zur Herstellung von hochreinem Isobuten.

Das Sumpfprodukt (V) der Kolonne K2 enthält die Hochsieder C8-Kohlenwasserstoffe und 2-Methoxybutan sowie MTBE. Sollen in der Kolonne vornehmlich C8-Kohlenwasserstoffe, beispielsweise Diisobuten, abgetrennt werden, kann der MTBE-Gehalt im Sumpfprodukt auf Werte kleiner 25 Massen-% reduziert werden. Soll zusätzlich 2-Methoxybutan abgetrennt werden, wird man aufgrund der kleinen Siedepunktsunterschiede zwischen 2-Methoxybutan und MTBE zweckmäßigerweise einen höheren MTBE-Gehalt im Sumpfprodukt zwischen 60 und 85 Massen-% zulassen, um den Aufwand für die Trennung zu reduzieren. In beiden Fällen kann dieses Gemisch thermisch verwertet werden, als Einsatzstoff für eine Synthesegasanlage genutzt werden oder direkt oder nach Hydrierung als Treibstoffkomponente verwendet werden.

### Verfahrensschritt d): MTBE-Spaltung

In Schritt d) des erfindungsgemäßen Verfahrens wird der in Verfahrensschritt b) gewonnene MTBE-Strom (IV), ggf. nach vorheriger Abtrennung von höhersiedenenden Komponenten in Verfahrenssschritt c) als Strom (VI), in einem Reaktor oder mehreren Reaktoren zu Isobuten und Methanol gespalten. Die MTBE-Spaltung erfolgt bevorzugt in der Gasphase an einem heterogenen Katalysator. Dabei können alle festen Katalysatoren eingesetzt werden, die im Temperaturbereich 150 bis 500 °C, insbesondere im Bereich von 200 bis 400 °C, die Spaltung von MTBE zu Isobuten und Methanol bewirken.

Die im erfindungsgemäßen Verfahren eingesetzten Katalysatoren können z. B. Metalloxide, Metallmischoxide, insbesondere solche, die Siliziumoxid und/oder Aluminiumoxid enthalten, Säuren auf Metalloxidträgern oder Metallsalze oder Mischungen davon sein.

Im erfindungsgemäßen Verfahren werden zur Spaltung von MTBE zu Isobuten und Methanol in der Gasphase bevorzugt Katalysatoren verwendet, die formal aus Magnesiumoxid, Aluminiumoxid und Siliziumoxid bestehen. Solche Katalysatoren werden z. B. in US 5 171 920 in Beispiel 4 oder in EP 0 589 557 beschrieben.

Besonders bevorzugt werden Katalysatoren eingesetzt, die formal Magnesiumoxid, Aluminiumoxid und Siliziumdioxid aufweisen, und die einen Anteil an Magnesiumoxid von 0,5 bis 20 Massen-%, bevorzugt von 5 bis 15 Massen-% und besonders bevorzugt von 10 bis 15 Massen-%, einen Anteil an Aluminiumoxid von 4 bis 30 Massen-%, bevorzugt von 10 bis 20 Massen-% und einen Anteil an Siliziumdioxid von 60 bis 95 Massen-%, bevorzugt von 70 bis 90 Massen-% aufweisen. Es kann vorteilhaft sein, wenn der Katalysator neben dem Magnesiumoxid ein Alkalimetalloxid aufweist. Dieses kann z. B. ausgewählt sein aus Na₂O oder K₂O. Vorzugsweise weist der Katalysator als Alkalimetalloxid Na₂O auf. Der bevorzugt eingesetzte Katalysator weist vorzugsweise eine BET-Oberfläche (volumetrisch bestimmt mit Stickstoff gemäß DIN ISO 9277) von 200 bis 450 m²/g, bevorzugt von 200 bis 350 m²/g auf. Wird der Katalysator als aktive Masse auf einem Träger aufgebracht, so weist nur die aktive Masse eine BET-Oberfläche im genannten Bereich auf. Das Material aus Katalysator und Träger kann dagegen je nach Beschaffenheit des Trägers eine deutlich abweichende BET-Oberfläche, insbesondere eine kleinere BET-Oberfläche aufweisen.

Das Porenvolumen des Katalysators beträgt vorzugsweise von 0,5 bis 1,3 ml/g, bevorzugt von 0,65 bis 1,1 ml/g.

Der mittlere Porendurchmesser gemäß DIN 66133 des Katalysators beträgt vorzugsweise von 5 bis 20 nm, bevorzugt von 8 bis 15 nm. Besonders bevorzugt entfällt mindestens 50 Massen-%, vorzugsweise über 70 Massen-% des Gesamtporenvolumens (Summe des Porenvolumens der Poren mit einem Porendurchmesser von größer gleich 3,5 nm bestimmt mit Quecksilberporosimetrie gemäß DIN 66133) des Katalysators auf Poren mit einem Durchmesser von 3,5 bis 50 nm (Mesoporen).

Im erfindungsgemäßen Verfahren werden bevorzugt Katalysatoren eingesetzt, die eine mittlere Korngröße (bestimmt durch Siebanalyse) von 10 µm bis 10 mm, vorzugsweise 0,5 mm bis 10 mm, besonders bevorzugt eine mittlere Korngröße von 1 bis 5 mm aufweisen. Vorzugsweise werden feste Katalysatoren eingesetzt, die eine mittlere Korngröße d₅₀, von 2 bis 4 mm, insbesondere von 3 bis 4 mm aufweisen.

In dem erfindungsgemäßen Verfahren kann der Katalysator als Formkörper eingesetzt werden. Die Formkörper können jegliche Form einnehmen. Bevorzugt wird der Katalysator als Formköper in Form von Kugeln, Extrudaten oder Tabletten eingesetzt. Die Formkörper weisen vorzugsweise die oben genannten mittleren Korngrößen auf.

Die Herstellung und Verwendung solcher Magnesium-Alumosilikat-Katalysatoren ist in DE 10 2006 040432 beschrieben. Es wird darauf Bezug genommen.

Die Spaltung des MTBE erfolgt in der Gasphase im Temperaturbereich von 150 bis 500 °C, insbesondere 200 bis 400 °C bei Drücken von 0,05 bis 2 MPa, insbesondere bei Drücken von 0,3 bis 1 MPa, ganz besonders bei Drücken von 0,5 bis 0,7 MPa.

Die Spaltung von MTBE in Isobuten und Methanol ist eine endotherme Reaktion. Um eine Partialkondensation von MTBE und Produkten zu vermeiden, ist es zweckmäßig, den Reaktor derart zu betreiben, dass die Minimaltemperatur im Reaktor größer als 150 °C, bevorzugt größer 200 °C ist. Daher liegt die Eingangstemperatur des MTBE, die mittels einem dem Reaktor vorgeschalteten Aufheizer eingestellt werden kann, mindestens bei 150 °C, vorzugsweise mindestens bei 200 °C.

Im Laufe des Betriebes kann es vorteilhaft sein, mit zunehmender Desaktivierung des Katalysators zur Konstanthaltung des Umsatzes die Eingangs- und/oder Betriebstempstemperatur bis auf 500 °C anzuheben. Kann der Umsatz bei Erreichen von 500 °C nicht mehr gehalten werden, so kann es vorteilhaft sein, den Katalysator ganz oder teilweise zu ersetzen.

Der Umsatz des MTBEs in Schritt d) des erfindungsgemäßen Verfahrens beträgt zwischen 40 % und 99 %, bevorzugt zwischen 70 % und 98 %, besonders bevorzugt zwischen 85 % und 95 %.

Der Reaktor wird vorzugsweise mit einer Raumgeschwindigkeit (Weight Hourly Space Velocity (WHSV) in Kilogramm Edukt je Kilogramm Katalysator je Stunde) von 0,1 bis 5 h⁻¹, insbesondere von 1 bis 3 h⁻¹ im geraden Durchgang betrieben.

Als Reaktoren werden bevorzugt Rohrreaktoren oder Rohrbündelreaktoren, insbesondere solche mit inneren Rohrdurchmessern von 10 bis 60 mm, verwendet. Sie werden vorzugsweise so betrieben, wie in DE 10 2006 040433 beschrieben.

Bei der Spaltung von MTBE treten Nebenreaktionen auf. Diese sind entweder auf MTBE oder die Spaltprodukte Isobuten und Methanol zurückzuführen. Standardmäßig treten bei der MTBE Spaltung die Bildung von Dimethylether (DME) aus Methanol auf sowie die Bildung von C₈-Kohlenwasserstoffen durch Dimerisierung von Isobuten auf. Durch Reaktion von Isobuten mit Wasser kann es zudem zur Bildung von tert.-Butanol (TBA) kommen, umgekehrt kann im Reaktorzulauf enthaltenes TBA zu Wasser und Isobuten gespalten werden.

Zu den Nebenreaktionen kommen meisten noch parallel ablaufende Reaktionen hinzu, bei denen Verunreinigungen aus dem MTBE reagieren. Darunter fällt beispielsweise die Spaltung von im MTBE enthaltenen 2-Methoxybutan. Aus diesem können sich durch Abspaltung von Methanol 1-Buten und 2-Butene bilden. Aus im MTBE enthaltenen 3-Methoxy-1-buten oder 1-Methoxy-2-buten kann in der Spaltung 1,3-Butadien gebildet werden.

### Verfahrensschritt e) Aufreinigung der Spaltprodukte

Um das Spaltproduktgemisch weiter aufzuarbeiten kann es vorteilhaft sein, wenn das Spaltprodukt (VII) in einer weiteren Destillation in Verfahrensschritt d) in einen Isobuten aufweisenden Kopfstrom (IX) und einen nicht umgesetztes MTBE aufweisenden Sumpfstrom VII getrennt wird. Die destillative Auftrennung des Spaltprodukts (VII) in einen Isobuten aufweisenden Kopfstrom (IX) und einen nicht umgesetztes MTBE aufweisenden Sumpfstrom (VIII) gemäß Verfahrensschritt d) erfolgt vorzugsweise in zumindest einer Kolonne, bevorzugt in genau einer Destillationskolonne K3.

Eine in Verfahrensschritt e) bevorzugt eingesetzte Destillationskolonne K3 weist vorzugsweise von 20 bis 55 theoretische Trennstufen, bevorzugt von 25 bis 50 und besonders bevorzugt von 30 bis 45 theoretische Trennstufen auf. Das Rücklaufverhältnis beträgt, in Abhängigkeit von der realisierten Stufenzahl, der Zusammensetzung des Reaktoraustrags und der erforderlichen Reinheiten von Destillat und Sumpfprodukt, vorzugsweise kleiner 5, bevorzugt kleiner 1. Der Betriebsdruck der Kolonne K3 kann vorzugsweise zwischen 0,1 und 2,0 MPa(abs) eingestellt werden. Um einen Kompressor zu sparen, kann es vorteilhaft sein, die Kolonne bei einem niedrigeren Druck als den Druck, mit dem der Spaltreaktor R in Verfahrensschritt b) betrieben wird, zu betreiben. Um Isobuten gegen Kühlwasser kondensieren zu können, ist ein Druck von ca. 0,5 MPa(abs) notwendig. Wird der Spaltung in Verfahrensschritt b) beispielsweise bei einem Druck von 0,65 MPa (abs) betrieben, kann es vorteilhaft sein, wenn die Destillationskolonne des Verfahrensschrittes c) mit einem Betriebsdruck von 0,55 bis 0,6 MPa (abs) durchgeführt wird. Zur Beheizung des Verdampfers kann z. B. 0,4 MPa-Dampf eingesetzt werden. Das Sumpfprodukt (VIII) enthält vorzugsweise nicht umgesetztes MTBE, Methanol sowie gegebenenfalls Nebenprodukte, wie beispielsweise Diisobuten, und 2-Methoxybutan. Das Kopfprodukt ist vorzugsweise Isobuten mit einer Reinheit größer 95 Massen-%, bezogen auf das gesamte Kopfprodukt.

Das in Verfahrensschritt e) erhaltene Sumpfprodukt (VIII) enthält das im Verfahrensschritt d) nicht umgesetzte MTBE, und den größten Teil des bei der Spaltung des MTBE entstandenen Methanols. Gegebenenfalls kann das Sumpfprodukt Nebenprodukte, wie beispielsweise Diisobuten und/oder 2-Methoxybutan enthalten. Für die Verwendung bzw. Aufarbeitung dieses Stroms (VIII) gibt es verschiedene Möglichkeiten. Steht die MTBE-Spaltanlage im Verbund mit einer Anlage zur Herstellung von MTBE, so kann Strom (VIII) in die MTBE Anlage, vorzugsweise in den Syntheseteil, gefahren werden. Dies gilt selbst dann, wenn die MTBE-Anlage nur gerade so viel MTBE erzeugt, wie für die Spaltung benötigt wird und somit im Syntheseteil kein weiterer Auslass für schwersiedende Komponenten vorhanden ist. Eine zweite Möglichkeit besteht darin, aus dem Strom VIII den größten Teil des Methanols destillativ abzutrennen und den Rest in den Verfahrensschritt b) zurückzufahren. Die letztere Möglichkeiten ist insbesondere für allein stehende Anlagen (stand alone plants), bei denen angeliefertes MTBE eingesetzt wird, vorteilhaft.

Aceton wird in der Destillationskolonne K3 anteilig ins Sumpfprodukt (VIII) abgetrennt, nur geringe Anteile des im Zulaufstrom (VII) enthaltenen Acetons gelangen ins Destillat (IX). Wird jedoch, wie zuvor beschrieben, der Sumpfstrom (VIII) vor den Reaktionsteil R zurückgefahren, reichert sich Aceton ohne die erfindungsgemäße Abtrennung in Verfahrensschritt b) auf unerwünscht hohe Konzentrationen an. Bevorzugt werden zwischen 90 und 99 Massen-% des im Reaktoraustrags (VII) enthaltenen Acetons über das Sumpfprodukt (VIII) abgetrennt. Bei fehlender Ausschleusung von Aceton in Verfahrensschritt b) bedeutet dies aber, dass sich Aceton im Kreis auf derart hohe Konzentrationen anreichert, dass nahezu die gesamte Menge des in Strom (I) enthaltenen Acetons ins Kopfprodukt (IX) der Kolonne K3 gelangt. Dies wird durch die erfindungsgemäße Ausschleusung in Verfahrensschritt b) verhindert.

Das Kopfprodukt (IX), das in Verfahrensschritt d) erhalten wird, erfindungsgemäß nahezu frei an Aceton ist und das vorzugsweise zu größer 95 Massen-% aus Isobuten besteht, kann direkt als Verkaufsprodukt eingesetzt werden oder weiter aufgereinigt werden.

Da Isobuten mit Methanol ein Minimum-Azeotrop bildet, kann das in Verfahrensschritt e) erhaltene Kopfprodukt (IX) neben dem Hauptprodukt Isobuten insbesondere Methanol enthalten. Als weitere Komponenten können im Kopfprodukt (IX) z. B. Dimethylether, welches z. B. durch Kondensation von Methanol entstanden sein kann, und lineare Butene (1-Buten, cis-2-Buten, trans-2-Buten), welches z. B. durch Zersetzung von 2-Methoxybutan entstanden sein können, und Wasser enthalten sein.

### Verfahrensschritt f): Isobutenaufarbeitung

Marktgängige Isobutenqualitäten sind üblicherweise praktisch frei von Methanol, siehe Tabelle 1. Das Methanol kann aus dem in Verfahrensschritt e) erhaltenen Strom (IX) nach an sich bekannten Verfahren, beispielsweise durch Extraktion, abgetrennt werden. Die Extraktion von Methanol aus Strom (IX) kann beispielsweise mit Wasser oder einer wässrigen Lösung als Extraktionsmittel z. B. in einer Extraktionskolonne durchgeführt werden. Vorzugsweise wird die Extraktion mit Wasser oder einer wässrigen Lösung in einer Extraktionskolonne K4 durchgeführt, die vorzugsweise von 4 bis 16 theoretische Trennstufen aufweist. Das Extraktionsmittel (XIII) kann in Bezug auf den zu extrahierenden Strom die Extraktionskolonne im Gleich- oder Gegenstrom durchströmen. Vorzugsweise wird das Extraktionsmittel (XIII) in Bezug auf den zu extrahierenden Strom im Gegenstrom durch die Extraktionskolonne geführt. Die Extraktion wird vorzugsweise bei einer Temperatur von 15 bis 50 °C, bevorzugt 25 bis 40 °C durchgeführt. Beispielweise kann bei der Verwendung einer Extraktionskolonne mit mehr als 6 theoretischen Trennstufen, die bei einem Druck von 0,9 MPa(abs) und einer Temperatur von 40°C betrieben wird, ein wassergesättigtes Isobuten mit einem Isobutengehalt von über 99 Massen-% erhalten werden.

Der bei der Extraktion erhaltene methanolhaltige Wasserextrakt (XI) kann destillativ in Wasser und Methanol getrennt werden. Bevorzugt erfolgt die Auftrennung in einer Destillationskolonne K5. Das Wasser kann als Extraktionsmittel (XIII) in die Extraktionsstufe zurückgeführt werden. Das Methanol (XII) kann für übliche technische Synthesen, beispielsweise Veresterungen oder Veretherungen genutzt werden. Bevorzugt wird das Methanol in die MTBE-Synthese zurückgeführt, aus der der Einsatzstrom (I) stammt.

Eventuell im isobutenhatigen Strom (IX) vorhandenes Aceton wird in der Extraktion aufgrund der Lösungsgleichgewichte nicht merklich abgetrennt und verbleibt anteilig im feuchten Isobutenstrom (X).

Der feuchte Isobutenstrom (X) aus der Extraktionskolonne kann in einer weiteren Destillationskolonne K6 von Dimethylether und Wasser getrennt und zu trockenem Isobuten aufgearbeitet werden. Das trockene Isobuten wird dabei als Sumpfprodukt (XVI) erhalten. Im Kondensationssystem am Kopf der Kolonne wird nach Phasentrennung Wasser (XIV) flüssig und Dimethylether (XV) gasförmig abgezogen. Eine für die Trocknung bevorzugt eingesetzte Destillationskolonne weist vorzugsweise von 30 bis 80 theoretische Trennstufen, bevorzugt von 40 bis 70 theoretische Trennstufen auf. Das Rücklaufverhältnis beträgt, in Abhängigkeit von der realisierten Stufenzahl und der erforderlichen Reinheit des Isobutens, vorzugsweise kleiner 100, bevorzugt kleiner 75. Der Betriebsdruck der Kolonne K2 kann vorzugsweise zwischen 0,1 und 2,0 MPa(abs) eingestellt werden.

Eventuell im Strom (X) vorhandenes Aceton wird in der Destillationskolonne K6 aufgrund der Siedepunktslage (siehe Tabelle 2) nicht merklich abgetrennt und verbleibt in diesem Fall anteilig im Isobutenprodukt (XVI).

Erfindungsgemäß wird aber Aceton in der Acetonabtrennung in Verfahrensschritt b) ausgeschleust, so dass erfindungsgemäß der Strom (X) frei von Aceton ist. Das derart gewonnene Isobuten kann z. B. die in Tabelle 1 aufgeführte Zusammensetzung aufweisen: Je nach Reinheitsanforderungen sind aber bei Bedarf auch geringere Konzentrationen der Nebenkomponenten denkbar.

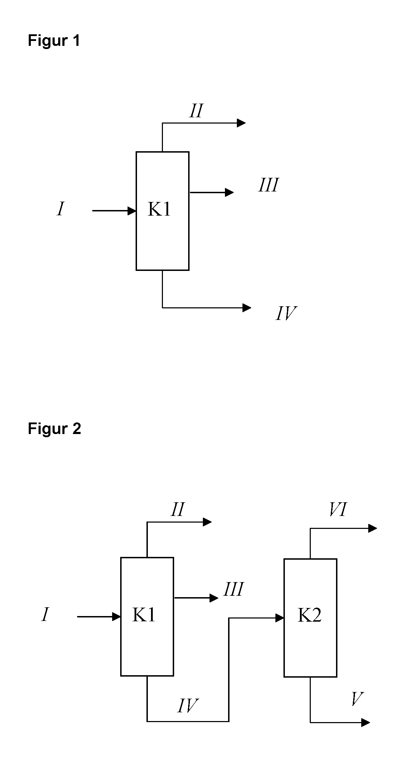

Ein Blockschema einer bevorzugten Ausführungsform, mit der das erfindungsgemäße Verfahren durchgeführt werden kann, ist in Figur 1 dargestellt. Das Einsatz MTBE (I) wird der Kolonne K1 zugeführt. In der Kolonne K1 wird das MTBE-haltige Gemisch (I) in ein Kopfprodukt (II), das vorwiegend die C4-und C5-Kohlenwasserstoffe enthält, einen Seitenstrom (III), der Aceton, Methanol und MTBE enthält, und einen Sumpfstrom (IV), der MTBE enthält, aufgetrennt wird. Im Sumpfstrom (IV) ist bevorzugt weniger als 50 Massen-% des im Einsatzstrom (I) enthaltenen Acetons enthalten.

Eine weitere bevorzugte Ausführungsform des Verfahrens ist in Figur 2 dargestellt. Dabei erfolgt die Abtrennung in Strom (I) des enthaltenen Acetons, Methanols und der enthaltenen Leichtsieder, insbesondere C4-und C5-Kohlenwasserstoffe, analog zu Figur 1 in der Kolonne K1. Der Sumpfstrom (IV) der Kolonne K2 wird in die Kolonne K2 geleitet. Dort werden enthaltene Hochsieder (C8-Kohlenwasserstoffe wie beispielsweise Diisobuten, 2-Methoxybutan) zumindest teilweise als Sumpfprodukt (V) abgetrennt. Das Kopfprodukt (VI) ist ein von Leichtsiedern, Methanol, Aceton und Hochsiedern weitgehend befreites MTBE und kann wegen seiner Reinheit im Pharmabereich und in der Analytik als Löse- und Extraktionsmittel eingesetzt werden. Insbesondere ist das Kopfprodukt (VI) auch als Einsatzstoff für die Herstellung von hochreinem Isobuten durch Rückspaltung von MTBE in Isobuten und Methanol geeignet.

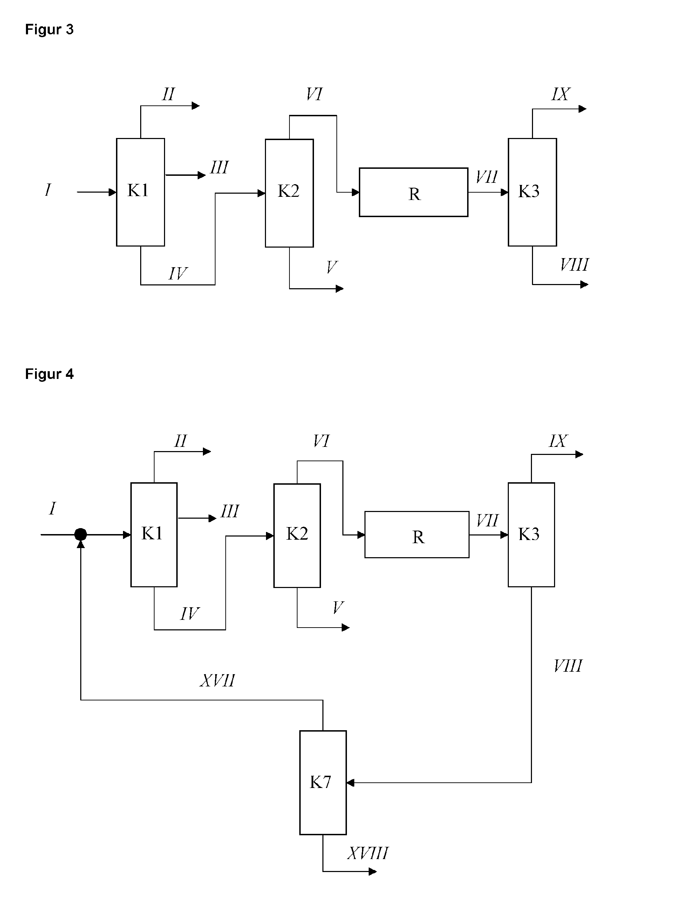

Ein derartiges Verfahren zur Herstellung von Isobuten durch Rückspaltung von MTBE ist in Figur 3 dargestellt. Bei dem Verfahren gemäß Figur 3 handelt es sich um eine weitere bevorzugte Ausführungsform des Verfahrens. Die Aufarbeitung des Einsatz-MTBEs (I) erfolgt analog zu Figur 2 in den Kolonne K1 und K2. Das Kopfprodukt (VI) der Kolonne K2 wird in den Spaltungsreaktor R geführt. Das Spaltungsprodukt (VII) wird in der Kolonne K3 in ein Kopfprodukt (IX), dass das gebildete Isobuten, DME und aufgrund derAzeotropbildung zwischen Isobuten und Methanol Anteile an Methanol enthält, und in ein Sumpfprodukt (VIII) mit dem nicht umgesetzten MTBE und den größeren Teil des entstandenen Methanols getrennt. Erfindungsgemäß wird Aceton anteilig in der Kolonne K3 über Sumpf abgetrennt. Erfindungsgemäß ist im Strom (VII) nur so wenig Aceton enthalten, dass das ins Kopfprodukt gelangte Aceton die Isobutenspezifikation nicht gefährdet. Das Sumpfprodukt (VIII) kann optional, nach Abtrennung von Methanol, vor die Kolonne K1 zurückgeführt werden, in eine MTBE-Synthese S1 zurückgeführt werden oder anderweitig verwendet werden.

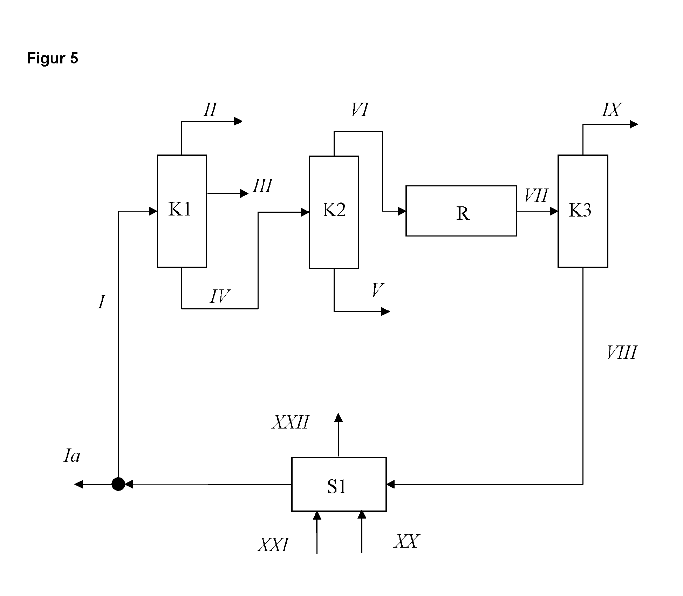

Bevorzugte Ausführungsformen zur Rückführung des Sumpfstroms (VIII) der Kolonne K3 sind in den Figuren 4 und 5 dargestellt.

In Figur 4 ist eine bevorzugte Ausführungsform des Verfahrens dargstellt, bei der der Sumpfstrom (VIII) der Kolonne K3 nach Abtrennung von Methanol vor die Kolonne K1 zurückgeführt wird. Aus dem Strom (VIII) wird in der Kolonne K7 der größte Teil des Methanols als Sumpfprodukt (XVIII) abgetrennt. Das Kopfprodukt (XVII), das MTBE und Teile von Methanol enthält, wird der Kolonne K1 zugeführt. In der Kolonne K7 verbleibt der überwiegende Teil des im Strom (VIII) enthaltenen Acetons im Kopfprodukt und wird erfindungsgemäß in der Kolonne K1 im Seitenabzug (III) ausgeschleust.

Figur 5 enthält eine bevorzugte Ausführungsform des Verfahrens, bei der der Sumpfstrom (VIII) der Kolonne K3 in eine MTBE-Synthese S1 zurückgeführt wird. Der MTBE-Synthese S1 werden mindestens ein isobutenhaltiger C4-Kohlenwasserstoffstrom (XX) und ein methanolhaltiger Strom (XXI) zugeführt. Der Strom (XXI) kann beispielsweise frisches Methanol enthalten oder Methanol, welches bei der Extraktion von Methanol-haltigen C4-Strömen mit Wasser und anschließender Methanol/Wasser-Trennung innerhalb des Verfahrens zurückgewonnen wurde. Das in Strom (XXI) enthaltene Methanol wird mit dem in (XX) enthaltenen Isobuten wird in der MTBE-Synthese S1 zu MTBE umgesetzt. Dieses MTBE wird direkt oder nach destillativer Abtrennung eines Stroms (XXII) als MTBE-haltiger Strom (I) in Schritt a) des erfindungsgemäßen Verfahrens eingesetzt. Wird vor dem Einsatz in Schritt a) ein Strom (XXII) abgetrennt, enthält dieser mindestens den größten Teil der die nicht umgesetzten C4-Kohlenwasserstoffe und auch noch Anteile an Methanol. Diese Ausführungsform kann insbesondere dann vorteilhaft sein, wenn die destillative Abtrennung des Stroms (XXII) in Form einer Reaktivdestillationskolonne ausgeführt wird. Der Einsatz von Reaktivdestillationskolonnen bei der MTBE Synthese ist Stand der Technik und wird verwendet um den Umsatz von Isobuten zu MTBE zu erhöhen.
Der MTBE-haltige Strom (I) wird wie beschrieben der Kolonne K1 zugeführt. Optional kann ein Strom (Ia) ausgeschleust und z.B. als technisches MTBE vermarktet werden. In dieser bevorzugten Ausführungsform gelangt im Strom (XX) enthaltens Aceton anteilig in Strom (I) und wird über Strom (III) aus dem Verfahrens ausgeschleust. Die Konzentration an Aceton im Kreislauf (Ströme (IV), (VI), (VII) und (VIII)) ist dabei so gering, dass Aceton nicht nennenswert in das Isobutenprodukt (IX) gelangt.

Je nach Wassergehalt der Ströme (XX) und (XXI) muss bei der Verfahrensvariante gemäß Figur 5 Wasser aus dem Verfahren ausgeschleust werden. Bevorzugte Varianten zur Wasserausschleusung sind in DE 10 2009 027404 beschrieben.

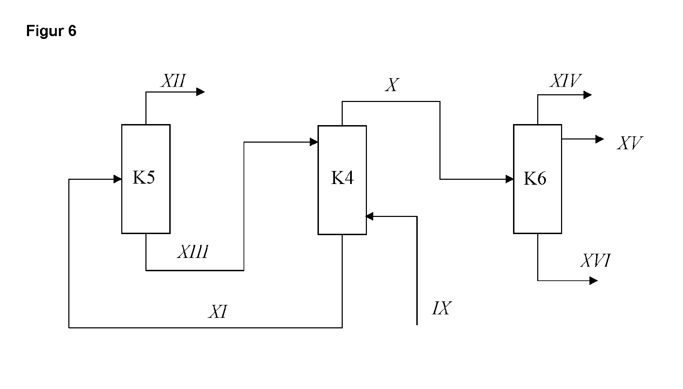

Figur 6 zeigt eine bevorzugte Ausführungsform für die weitere Aufarbeitung des in Verfahrensschritt e) erhaltenen methanolhaltigen Isobutenstroms (IX). Aus dem Strom (IX) wird in einer Extraktionskolonne K4 Methanol mit Wasser (XIII) ausgewaschen. Das methanolbeladene Wasser (XI) wird in der Kolonne K5 in einen methanolhaltigen Strom (XII) und Wasser (XIII) getrennt, das Wasser zurück in die Extraktion geführt. Der Strom (XII) wird bevorzugt in eine MTBE-Synthese zurückgefahren, besonders bevorzugt in die MTBE-Synthese, aus der der Strom (I) entstammt. Der wassergesättigte Isobutenstrom (X), der noch DME enthält, wird anschließend in der Kolonne K6 weiter aufgereinigt. Am Kopf der Kolonne wird ein DME-haltiger Strom (XIV) erhalten, der normalerweise noch Isobuten enthält. Zur Abtrennung des Wassers ist es sinnvoll, die Kolonne K6 mit einem Kopfdekanter auszustatten, in dem sich das Wasser (XV) als zweite Phase abscheidet und ausgeschleust werden kann. Im Sumpf der Kolonne wird Isobuten (XV) erhalten. Im Strom (IX) eventuell vorhandenes Aceton wird weder in der Kolonne K4 noch in der Kolonne K6 vollständig abgetrennt und gelangt daher zum größten Teil ins Isobutenprodukt (XVI). Erfindungsgemäß ist der Strom (IX) aber weitestgehend frei von Aceton, so dass auch Strom (XVI) weitestgehend frei von Aceton ist.

Werden in dem erfindungsgemäßen Verfahren Kolonnen eingesetzt, wie beispielsweise die in den Figuren 1 bis 6 mit K1 bis K7 bezeichneten Kolonnen, so können diese mit Einbauten, die z. B. aus Böden, rotierenden Einbauten, regellosen Schüttungen und/oder geordneten Packungen sind, versehen sein.

Bei den Kolonnenböden können z. B. folgende Typen zum Einsatz kommen:
- Böden mit Bohrungen oder Schlitzen in der Bodenplatte.
- Böden mit Hälsen oder Kaminen, die von Glocken, Kappen oder Hauben überdeckt sind.
- Böden mit Bohrungen in der Bodenplatte, die von beweglichen Ventilen überdeckt sind.
- Böden mit Sonderkonstruktionen.

Bei Einsatz von Kolonnen mit regellosen Schüttungen mit verschiedenen Füllkörpern können die Füllkörper aus fast allen Werkstoffen, insbesondere aus Stahl, Edelstahl, Kupfer, Kohlenstoff, Steingut, Porzellan, Glas oder Kunststoffen bestehen und die verschiedensten Formen, insbesondere die Form von Kugeln, Ringen mit glatten oder profilierten Oberflächen, Ringen mit Innenstegen oder Wanddurchbrüchen, Drahtnetzringen, Sattelkörper und Spiralen aufweisen.

Packungen mit regelmäßiger/geordneter Geometrie können z. B. aus Blechen oder Geweben bestehen. Beispiele für solche Packungen sind Sulzer Gewebepackungen BX aus Metall oder Kunststoff, Sulzer Lamellenpackungen Mellapak aus Metallblech, Hochleistungspackungen von Sulzer wie Mella-pakPlus, Strukturpackungen von Sulzer (Optiflow), Montz (BSH) und Kühni (Rombopak).

Das mit dem erfindungsgemäßen Verfahren hergestellte Isobuten kann z. B. zur Herstellung von Methacrylsäure, Methylmethacrylat, Diisobuten, Polyisobuten, Alkylphenolen, Methallylchlorid, oder Methallylsulfonaten eingesetzt werden. Insbesondere kann es vorteilhaft sein, sowohl das bei der Spaltung erhaltene Methanol als auch das Isobuten zur Herstellung von Methylmethacrylat einzusetzen. Ein solches Verfahren zur Herstellung von Methylmethacrylat wird beispielsweise in EP 1 254 887 beschrieben, auf welches ausdrücklich verwiesen wird.

Die folgenden Beispiele sollen die Erfindung erläutern.

### Beispiele

### Beispiel 1:

Die MTBE-Spaltung wurde in einem Rohrreaktor mit Heizmantel, durch den ein Wärmeträgeröl (Marlotherm SH der Sasol Olefins & Surfactans GmbH) floss, durchgeführt. Als Katalysator wurde ein mit Magnesium dotiertes Silika-Alumina verwendet. Die Herstellung des Katalysators erfolgte entsprechend der Patentanmeldung DE 10 2006 040432, s. Beispiel 2. Als Edukt wurde MTBE hoher Reinheit eingesetzt, welches normalerweise nicht als Kraftstoffzusatz sondern als Lösungsmittel eingesetzt wird (DRIVERON-S der Firma Evonik Oxeno GmbH).

Vor Eintritt in den Reaktor wurde das MTBE in einem Verdampfer bei 260 °C vollständig verdampft. Die Spaltung wurde bei einer Temperatur von 261 °C (Temperatur des Marlotherms im Zulauf des Reaktormantels) durchgeführt, der Druck wurde über eine Druckhaltung am Ende des Reaktors auf konstant 0,7 MPa_{(abs)} eingestellt. Der MTBE Zulauf wurde auf konstant 1500 g/h eingeregelt, was bei einer Katalysatormenge von 331 g einem WHSV Wert von 4,53 h⁻¹ entspricht. Das den Reaktor verlassende gasförmige Spaltgemisch wurde vollständig kondensiert und gaschromatographisch analysiert.

Nach einer Standzeit von 1600 Stunden betrug der Umsatz des MTBEs unter den gewählten Reaktionsbedingungen 90,6 %, die Selektivität zu Dimethylether betrug 3,53 % und die Selektivität zu Diisobuten betrug 0,05 %. Nach 1613 Stunden wurde Aceton zum Reaktorzulauf zudosiert, der Reaktorzulauf und der Reaktoraustrag wurde mittels GC-Analyse auf Aceton hin untersucht. Die Ergebnisse zeigt Tabelle 4.

**Tabelle 4: Analysen Reaktorzulauf, Reaktoraustrag (jeweils Massenanteile)**

| Zeit | Aceton-Konz. im Zulauf | Aceton-Konz. im Austrag |
|---|---|---|
| [h] | [Massen-ppm] | [Massen-ppm] |
| 1613 | 342 | 320 |
| 1627 | 320 | 315 |
| 1681 | 300 | 298 |
| 1705 | 311 | 310 |
| 1729 | 258 | 253 |
| 1753 | 299 | 280 |
| 1797 | 314 | 320 |
| 1837 | 314 | 325 |
| 1849 | 310 | 305 |

Es konnte keine merkliche Abnahme an Aceton festgestellt werden, die Wiederfindungsrate liegt in alle Fällen oberhalb von 90 Massen-% und damit im Rahmen der Messgenauigkeit. Während der Dosierung von Aceton konnten in Spuren Methylvinylketon (bis 8 ppm) und 2-Methoxypropen (bis 2 ppm) nachgewiesen werden.

Umsatz an MTBE und Selektivitäten zu Dimethylether und Diisobuten blieben unverändert.

Mit diesem Beispiel konnte gezeigt werden, dass Aceton im Reaktionsteil der MTBE-Spaltung nicht merklich abgebaut wird und damit bei Anwesenheit im Reaktorzulauf in nahezu unveränderter Konzentration auch im Austrag des Reaktionsteils vorhanden ist.

### Erläuterungen zu den Beispielen 2, 3, 4 und 5

In den nachfolgenden Beispielen 2, 3, 4 und 5 wurden Rechnungen mit dem stationären Simulationsprogramm ASPEN Plus (Version 2006 der Firma AspenTech) durchgeführt, die die Erfindung weiter erläutern und die Auswirkungen der Acetonausschleusung auf den Gesamtprozess einer MTBE-Spaltung wiedergeben.

Um transparente, reproduzierbare Daten zu erzeugen, wurden nur allgemein zugängliche Stoffdaten eingesetzt. In den Beispielen die Property-Methode "NRTL-RK" (s. H. Renon and J.M. Prausnitz, "Local Compositions in Thermodynamic Excess Functions for Liquid Mixtures," AlChE J., Vol. 14, No. 1, (1968), pp. 135- 144 und O. Redlich and J.N.S. Kwong, "On the Thermodynamics of Solutions V. An Equation-of-state. Fugacities of Gaseous Solutions," Chem. Rev., Vol. 44, (1979), pp. 223 - 244) benutzt.

Für die Modellierung der Reaktoren RS1-1, RS1-2 und RS1-3 in der MTBE-Synthese sowie des Spaltungsreaktors R in der MTBE-Spaltung wurden in den Rechnungen kinetische Reaktormodelle verwendet, die auf umfangreichen experimentellen Messdaten mit den jeweiligen Katalysatoren beruhen. In den Beispielen werden daher jeweils auch die Reaktionstemperaturen genannt, die bei der Reaktormodellierung angenommen wurden. Da auch jeweils die Zusammensetzung der ein- und ausgehenden Ströme der Reaktionsstufen genannt werden, ist es dem Fachmann durch Nachstellung der Reaktoren mit fest vorgegebenen Umsätzen möglich, das Beispiel nachzurechnen, ohne die genauen Gleichungen für die Kinetik zu kennen.

### Beispiel 2a: (nicht erfindungsgemäß)

In Beispiel 2a wird eine Kolonne K1* betrachtet, in der MTBE-haltige Einsatzstrom (I) lediglich von Leichtsiedern wie C4- und C5-Kohlenwasserstoffe befreit wird. Eine deartige Kolonne ist in den meisten Verfahren, die aus der Literatur bekannt sind, beschrieben, z.B. DE 10 2009 027404. Demnach wird in der Kolonne K1* der MTBE-haltige Einsatzstoff (I) in ein Kopfprodukt (II*) und ein Sumpfprodukt (IV*) getrennt. Als Zulauf zu der Kolonne K1* wird ein MTBE-Strom (I) (MTBE-haltige Einsatzstrom) von 1000 kg/h mit der in Tabelle 5 aufgeführten Zusammensetzung angenommen (typisches Kraftstoff-MTBE, vergleiche mit Tabelle 3).

**Tabelle 5: Zusammensetzung des angenommen MTBE-Eintrittstroms (I) sowie des Destillats (II*) und des Sumpfsprodukts (IV*) für Kolonne K1* für Beispiel 2a**

| | Einsatz-MTBE (I) | Destillat K1* (II*) | Sumpfprod. K1* (IV*) |
|---|---|---|---|
| Massenstrom [kg/h] | 1000,0 | 4,2 | 995,8 |
| Massenanteile [kg/kg] | | | |
| Dimethylether | | | |
| Isobuten | | | |
| n-Butan | 0,000150 | 0,035549 | |
| 1-/2-Butene | 0,001030 | 0,244102 | |
| C5-Kohlenwasserstoffe | 0,002000 | 0,462191 | 0,000050 |
| MTBE | 0,979165 | 0,150000 | 0,982679 |
| 2-Methoxybutan | 0,002500 | 0,000064 | 0,002510 |
| Methanol | 0,009500 | 0,108011 | 0,009083 |
| Tert.-Butanol | 0,003000 | | 0,003013 |
| Wasser | 0,000005 | 0,000005 | 0,000005 |
| Diisobuten | 0,002500 | | 0,002511 |
| Aceton | 0,000150 | 0,000078 | 0,000150 |

Die Kolonne K1* hat 40 theoretische Stufen und wird bei einem Druck von 0,35 MPa_{(abs)} betrieben. Die Aufgabe des Zulaufs (I) erfolgt oberhalb Stufe 15, gezählt von oben. Der Rücklauf wurde mit 671 kg/h so eingestellt, dass zum einen der MTBE-Gehalt im Destillat (II*) gering ist (MTBE-Verlust), zum anderen das Sumpfprodukt (IV*) weitestgehend frei von Leichtsiedern (C4- und C5-Kohlenwasserstoffen) ist. Das Destillat dieser Kolonne K1* hat einen Restgehalt von 15 Massen-% MTBE und das Sumpfprodukt einen Restgehalt an C4- und C5-Kohlenwasserstoffen von 50 ppm. Das im Einsatz-MTBE enthaltene Aceton (150 ppm) wird in dieser Fahrweise der Kolonne nicht abgetrennt, die Konzentration im Sumpfprodukt (IV*) beträgt unverändert 150 ppm.

Mit diesem Beispiel konnte gezeigt werden, dass bei einer üblichen Fahrweise der in der Literatur beschriebenen Leichtsiederkolonne Aceton aus dem Einsatz-MTBE (I) nicht entfernt wird.

### Beispiel 2b: (nicht erfindungsgemäß)

In Beispiel 2b wird der Destillatmassenstrom der Kolonne K1* so eingestellt, dass bei gleichen Rücklaufstrom 80 Massen-% des im Zulaufstroms (I) enthaltenen Acetons ins Destillat abgetrennt werden. Massenstrom und Zusammensetzung des Zulaufstroms (I) sowie Druck, Stufenzahl und Aufgabestufe für Kolonne K1* bleiben gegenüber Beispiel 2a unverändert.

Die Massenströme und die entsprechenden Zusammensetzung von Destillat (II*) und Sumpfprodukt (IV*) zeigt Tabelle 6

**Tabelle 6: Zusammensetzung des Destillats (II*) und des Sumpfsprodukts (IV*) für Kolonne K1* für Beispiel 2b**

| | Destillat K1* (II*) | Sumpfprod. K1* (IV*) |
|---|---|---|
| Massenstrom [kg/h] | 232,9 | 767,1 |
| Massenanteile [kg/kg] | | |
| Dimethylether | | |
| Isobuten | | |
| n-Butan | 0,000644 | |
| 1-/2-Butene | 0,004423 | |
| C5-Kohlenwasserstoffe | 0,008570 | 0,000006 |
| MTBE | 0,942549 | 0,990281 |
| 2-Methoxybutan | 0,001066 | 0,002935 |
| Methanol | 0,040794 | |
| Tert.-Butanol | 0,001417 | 0,003480 |
| Wasser | 0,000021 | |
| Diisobuten | | 0,003259 |
| Aceton | 0,000515 | 0,000039 |

Wie aus Tabelle 6 ersichtlich, muss der Destillatmassenstrom von 4,2 kg/h in Beispiel 2a auf 233 kg/h erhöht werden, um 80 Massen-% des im Zulaufstrom (I) enthaltenden Acetons aus dem Sumpfprodukt (IV*) abzutrennen. Der MTBE-Gehalt im Destillat liegt bei ca. 94 Massen-%. Damit ist der MTBE-Verlust beträchtlich (22 Massen-%). Der Gehalt an C4- und C5-Kohlenwasserstoffen im Destillat liegt in Summe bei ca. 1,4 Massen-%. Damit ist dieser Strom, ohne erneute Aufarbeitung, nicht als Kraftstoffadditiv geeignet.

### Beispiel 2c: (nicht erfindungsgemäß)

In Beispiel 2c wird der Rücklaufstrom der Kolonne K1* auf 947 kg/h erhöht und der Destillatmassenstrom der Kolonne K1* so eingestellt, dass wieder 80 Massen-% des im Zulaufstroms (I) enthaltenen Acetons ins Destillat abgetrennt werden. Massenstrom und Zusammensetzung des Zulaufstroms (I) sowie Druck, Stufenzahl und Aufgabestufe für Kolonne K1* bleiben gegenüber Beispiel 2a unverändert.

Die Massenströme und die entsprechenden Zusammensetzung von Destillat (II*) und Sumpfprodukt (IV*) zeigt Tabelle 7

**Tabelle 7: Zusammensetzung des Destillats (II*) und des Sumpfsprodukts (IV*) für Kolonne K1* für Beispiel 2c**

| | Destillat K1* (II*) | Sumpfprod. K1* (IV*) |
|---|---|---|
| Massenstrom [kg/h] | 130,2 | 869,8 |
| Massenanteile [kg/kg] | | |
| Dimethylether | | |
| Isobuten | | |
| n-Butan | 0,001152 | |
| 1-/2-Butene | 0,007909 | |
| C5-Kohlenwasserstoffe | 0,015331 | 0,000004 |
| MTBE | 0,900536 | 0,990939 |
| 2-Methoxybutan | 0,000530 | 0,002795 |
| Methanol | 0,072946 | |
| Tert.-Butanol | 0,000638 | 0,003354 |
| Wasser | 0,000038 | |
| Diisobuten | | 0,002874 |
| Aceton | 0,000921 | 0,000034 |

Trotz Erhöhung des Rücklaufs werden noch immer ca. 11 Massen-% des im Zulaufstroms enthaltenen MTBEs über das Destillat ausgeschleust (MTBE-Verlust). Der Gehalt an C4- und C5-Kohlenwasserstoffen im Destillat (I*) hat sich gegenüber Beispiel 2b auf ca. 2,4 Massen-%. Damit ist dieser Strom, ohne erneute Aufarbeitung, nicht als Kraftstoffadditiv geeignet.

### Beispiel 3a: (erfindungsgemäß)

Das Beispiel 3a entspricht dem in Figur 1 dargestellten Verfahren. Die Kolonne K1 wird im Gegensatz zu der Kolonne K1* in Beispiel 2a und 2b mit einem Seitenabzug (III) betrieben. Massenstrom und Zusammensetzung des Zulaufstroms (I) bleiben gegenüber Beispiel 2a unverändert.

Die Kolonne K1 hat 52 theoretische Stufen und wird bei einem Druck von 0,35 MPa_{(abs)} betrieben. Die Aufgabe des Zulaufs (I) erfolgt oberhalb Stufe 27, gezählt von oben. Der Seitenabzug (II) wird von Stufe 12 flüssig entnommen und auf 70 kg/h eingestellt. Der Rücklauf wurde wie in Beispiel 2a (670 kg/h) gewählt.

**Tabelle 8: Zusammensetzung des Destillats (II), des Seitenabzugs (III) und des Sumpfsprodukts (IV) für Kolonne K1 für Besipiel 3a**

| | Destillat K1 (II) | Sumpfprod. K1 (IV) | Seitenabzug K1 (III) |
|---|---|---|---|
| Massenstrom [kg/h] | 4,0 | 926,0 | 70,0 |
| Massenanteile [kg/kg] | | | |
| Dimethylether | | | |
| Isobuten | | | |
| n-Butan | 0,036276 | | 0,000054 |
| 1-/2-Butene | 0,245209 | | 0,000593 |
| C5-Kohlenwasserstoffe | 0,460260 | 0,000023 | 0,001758 |
| MTBE | 0,150000 | 0,991320 | 0,866124 |
| 2-Methoxybutan | 0,000018 | 0,002670 | 0,000391 |
| Methanol | 0,107805 | | 0,129506 |
| Tert.-Butanol | | 0,003222 | 0,000235 |
| Wasser | 0,000009 | | 0,000071 |
| Diisobuten | | 0,002700 | |
| Aceton | 0,000424 | 0,000064 | 0,001267 |

Das Destillat (II) dieser Kolonne K1 hat, wie in Bespiel 2a, einen Restgehalt von 15 Massen-% MTBE, während das Sumpfprodukt (IV) nur noch 23 ppm an C5-Kohlenwasserstoffen enthält (siehe Tabelle 8). Das im Einsatz-MTBE enthaltene Aceton (150 ppm) wird in dieser Fahrweise zu ca. 60 Massen-% über den Seitenstrom (II) ausgeschleust, der Sumpfstrom (IV) enthält nur noch ca. 40 Massen-% der ursprünglichen Menge. Wie aus Tabelle 8 zu entnehmen, bleibt der Destillatmassenstrom (I) gegenüber Beispiel 2a nahezu unverändert, damit auch der MTBE-Verlust über diesen Strom. Im Seitenstrom werden nur ca. 6 Massen-% der im Zulaufstrom enthaltenen Menge an MTBE ausgeschleust. Im Gegensatz zu den Destillatströmen in Beispiel 2b und 2c ist der Gehalt an C4 und C5-Kohlenwasserstoffen im Seitenstrom aber gering (ca. 2400 ppm), so dass dieser Strom (II) als Kraftstoffadditiv vermarktet werden kann, ggf. auch nach Mischung mit einem Strom aus einer MTBE-Synthese.

### Beispiel 3b: (erfindungsgemäß)

In Beispiel 3b wird der Rücklaufstrom der Kolonne K1, wie in Beispiel 2c, auf 947 kg/h erhöht und der Destillatmassenstrom der Kolonne K1 so eingestellt, dass das Destillat (II) wieder einen Restgehalt von 15 Massen-% MTBE enthält. Zusammensetzung des Zulaufstroms (I) sowie Druck, Stufenzahl und Aufgabestufe für Zulaufstrom und Entnahmestufe für den Seitenabzug für Kolonne K1 bleiben gegenüber Beispiel 3a unverändert.

Die Massenströme und die entsprechenden Zusammensetzung von Destillat (II), Seitenabzug (III) und Sumpfprodukt (IV) zeigt Tabelle 9.

**Tabelle 9: Zusammensetzung des Destillats (II), des Seitenabzugs (III) und des Sumpfsprodukts (IV) für Kolonne K1 für Beispiel 3b**

| | Destillat K1 (II) | Sumpfprod. K1 (IV) | Seitenabzug K1 (III) |
|---|---|---|---|
| Massenstrom [kg/h] | 4,1 | 925,9 | 70,0 |
| Massenanteile [kg/kg] | | | |
| Dimethylether | | | |
| Isobuten | | | |
| n-Butan | 0,035756 | | 0,000038 |
| 1- / 2-Butene | 0,242768 | | 0,000426 |
| C5-KohlenwasserstofFe | 0,462660 | 0,000004 | 0,001295 |
| MTBE | 0,150000 | 0,991366 | 0,866584 |
| 2-Methoxybutan | 0,000015 | 0,002676 | 0,000323 |
| Methanol | 0,108231 | | 0,129344 |
| Tert.-Butanol | | 0,003222 | 0,000237 |
| Wasser | 0,000009 | | 0,000071 |
| Diisobuten | | 0,002700 | |
| Aceton | 0,000562 | 0,000032 | 0,001681 |

Durch die Erhöhung des Rücklaufs können in Beispiel 3b 80 Massen-% des im Zulaufstroms über Seitenabzug und Destillat abgetrennt werden, wobei der Seitenabzug ca. Massen-79 % des Acetons enthält. Im Gegensatz zu Beispiel 2 b und 2c ist aber der MTBE-Verlust über das Destillat (I) gering, und über den Seitenabzug werden wieder nur ca. Massen-6 % der im Zulaufstrom enthaltenen Menge an MTBE ausgeschleust. Gegenüber Beispiel 3a konnte durch Erhöhung der Rücklaufs der Gehalt an C4 und C5-Kohlenwasserstoffen im Seitenstrom weiter reduziert werden (ca. 1760 ppm), so dass dieser Strom (II) wieder als Kraftstoffadditiv vermarktet werden kann.

Mit den Beispielen 2a, 2b, 2c, 3a und 3b konnte gezeigt werden, dass bei einer üblichen Fahrweise der in der Literatur beschriebenen Leichtsiederkolonne K1* Aceton aus dem Einsatz-MTBE (I) nicht entfernt wird. Durch Erhöhung der Destillatmassenstroms (II)* und des Rücklaufs kann zwar mit der gleichen Konfiguration (Kolonne K1* ohne Seitenabzug) Aceton entfernt werden, jedoch ist mit dieser Fahrweise ein unerwünscht hoher MTBE-Verlust verbunden. Mit der erfindungsgemäßen Konfiguration der Leichtsieder Kolonne - Kolonne K1 mit Seitenabzug - kann Aceton effektiv und mit geringeren MTBE-Verlusten abgetrennt werden.

### Beispiel 4: (erfindungsgemäß)

Das Beispiel 4 entspricht dem in Figur 5 dargestellten Verfahren zu Herstellung von hochreinem Isobuten, wobei für die Aufreinigung des Isobutens (IX) eine Variante gemäß Figur 6 angenommen wird.

Als Zulauf zur MTBE-Synthese S1 wird gemäß Figur 5 ein C4-Kohlenwasserstoffstrom (XX) von 1800 kg/h mit der in Tabelle 10 aufgeführten Zusammensetzung mit einem Anteil von 60 ppm Aceton angenommen (typisches Raffinat I). Mit aufgeführt ist in Tabelle 10 ebenfalls die Zusammensetzung und der Massenstrom des zugeführten Frischmethanols (XX). Zusammensetzung und Massenstrom des aus Verfahrenschritt e) kommenden Rückführstroms (VIII) (Sumpfprodukt Kolonne K3) sind in Tabelle 13 dargestellt. Die Frischmethanolmenge wurde so eingestellt, dass sich ein molares Verhältnis von Methanol zu Isobuten im Zulauf zur MTBE-Synthese von 1,13 ergibt.

**Tabelle 10: Zusammensetzung des Eintrittsstroms (XX) und des Frischmethanols (XXI) sowie des Destillats (XII) und des Sumpfprodukts (I) (Einsatz-MTBE) der Kolonne KS1-1 für Beispiel 4**

| | Raffinat I (XX) | Methanol (XXI) | Destillat KS1-1 (XXII) | Einsatz-MTBE (I) |
|---|---|---|---|---|
| Massenstrom [kg/h] | 1800,0 | 120,1 | 1249,5 | 1117,1 |
| Massenanteile [kg/kg] | | | | |
| Dimethylether | | | 0,000709 | |
| Isobutan | 0,025500 | | 0,036736 | |
| Isobuten | 0,350000 | | 0,020956 | |
| n-Butan | 0,080000 | | 0,115250 | |
| Butadien | 0,001500 | | 0,002161 | |
| 1-/2-Butene | 0,542400 | | 0,779989 | 0,000441 |
| C5-Kohlenwasserstoffe | 0,000540 | | 0,000056 | 0,000808 |
| MTBE | | | | 0,972864 |
| 2-Methoxybutan | | | | 0,003335 |
| Methanol | | 1,000000 | 0,043415 | 0,009500 |
| Tert.-Butanol | | | | 0,011162 |
| Wasser | | | 0,000727 | 0,000003 |
| Diisobuten | | | | 0,001768 |
| Aceton | 0,000060 | | | 0,000119 |

Der C4-Kohlenwasserstoffstrom (XX), das Frischmethanol (XXI) und der methanolhaltige Rückführungsstrom (VIII) werden in Verfahrensschritt S1 vermischt und einer MTBE-Synthese zugeführt. Die MTBE-Synthese besteht in Beispiel 4 aus drei in Reihe geschalteten, adiabatischen Festbettreaktoren RS1-1, RS1-2 und RS1-3 und einer Destillationskolonne KS1-1. Der erste Reaktor RS1-1 ist als Kreislaufreaktor ausgeführt. Es wird eine Füllung der Reaktoren mit Amberlyst® 15 (Rohm & Haas) angenommen. Der Reaktor RS1-1 wird mit einem Volumen von 3,25 m³ modelliert, RS1-2 mit 1,8 m³ und RS1-3 mit 1 m³. Die Eintrittstemperatur in den Reaktor RS1-1 beträgt 42°C, der angenommene Kreislaufstrom 5100 kg/h. Die Eintrittstemperatur in den Reaktor RS1-2 beträgt 40°C, die Eintrittstemperatur in den Reaktor RS1-3 36°C. Unter diesen Bedingungen ergibt sich ein Umsatz von Isobuten über alle drei Reaktoren von ca. 96 %. Als Nebenreaktion werden im verwendeten kinetischen Modell die Bildung von TBA aus Isobuten und Wasser, die Dimerisierung von Isobuten zu Diisobuten, die Reaktion von Methanol zu DME und Wasser sowie die Bildung von 2-Methoxybutan aus n-Butenen berücksichtigt.

Der Reaktoraustrag aus dem Reaktor RS1-3 wird der Kolonne KS1-1 zugeführt. Die Kolonne hat 70 theoretische Stufen und der Zulauf erfolgt oberhalb Stufe 50, gezählt von oben. Die Kolonne wird bei einem Rücklaufverhältnis von 0,9 und bei einem Druck von 0,65 MPa_{(abs)} betrieben. Das Sumpfprodukt (I) (Einsatz-MTBE) stellt technisches MTBE da, siehe Tabelle 10. In der Kolonne KS1-1 gelangt Aceton komplett in den Sumpf. Das Destillat (XXII) besteht überwiegend aus C4-Kohlenwasserstoffen sowie aus Methanol, in der MTBE-Synthese gebildetem DME sowie Wasser. In weiteren, aus der Literatur bekannten Aufarbeitungsschritten, z.B. Extraktion, kann das Destillat (XXII) von Oxygenates befreit und zu weiteren Wertprodukten weiter verarbeitet werden.

Der MTBE-Strom (I) enthält in Summe noch ca. 1250 ppm C4- und C5-Kohlenwasserstoffe sowie ca. 120 ppm Aceton. Aus diesem Strom werden in der Kolonne K1 erfindungsgemäß die C4- und C5-Kohlenwasserstoffe sowie das Aceton abgetrennt. Die Kolonne K1 hat, wie in Bespiel 3a und 3b, 52 theoretische Stufen und wird bei einem Rücklauf von 1341 kg/h und bei einem Druck von 0,35 MPa_{(abs)} betrieben. Die Aufgabe des Zulaufs (I) erfolgt oberhalb Stufe 27, gezählt von oben. Der Seitenabzug (II) wird von Stufe 12 flüssig entnommen und auf 70 kg/h eingestellt.

Tabelle 11 zeigt die des Destillats (II), des Seitenabzugs (III) und des Sumpfsprodukts (IV) der Kolonne K1.

**Tabelle 11: Zusammensetzung des Destillats (II), des Seitenabzugs (III) und des Sumpfsprodukts (IV) der Kolonne K1 für Beispiel 4**

| | Destillat K1 (II) | Seitenabzug K1 (III) | Sumpfprod. K1 (IV) |
|---|---|---|---|
| Massenstrom [kg/h] | 1,8 | 70,0 | 1045,2 |
| Massenanteile [kg/kg] | | | |
| Dimethylether | | | |
| Isobutan | | | |
| Isobuten | | | |
| n-Butan | 0,000004 | | |
| Butadien | | | |
| 1-/2-Butene | 0,264378 | 0,000133 | |
| C5-Kohlenwasserstoffe | 0,476646 | 0,000424 | 0,00001 |
| MTBE | 0,150000 | 0,848364 | 0,982642 |
| 2-Methoxybutan | 0,000017 | 0,000369 | 0,003539 |
| Methanol | 0,108444 | 0,148768 | |
| Tert.-Butanol | | 0,000384 | 0,011903 |
| Wasser | 0,000005 | 0,000051 | |
| Diisobuten | | | 0,001889 |
| Aceton | 0,000506 | 0,001507 | 0,000025 |

Das Destillat der Kolonne K1 hat einen Restgehalt von 15 Massen-% MTBE. Der Seitenstrom enthält den überwiegenden Anteil des in Strom (I) enthaltenen Acetons (ca. 79 Massen-%), während das Sumpfprodukt (IV) erfindungsgemäß nur noch sehr wenig Aceton enthält (25 ppm, 20 Massen-% der in Strom (I) enthaltenen Menge).

Der von Leichtsiedern und Aceton befreite MTBE-Strom (IV) wird der Kolonne K2 zugeführt, in der vornehmlich Diisobuten und 2-Methoxybutan über Sumpf (V) abgetrennt werden. Die Kolonne hat 105 theoretische Stufen und wird bei einem Rücklaufverhältnis von 3,2 und bei einem Druck von 0,8 MPa(abs) betrieben. Die Zugabe von Strom (IV) erfolgt oberhalb Stufe 40, von oben gezählt. Als Kopfprodukt (VI) wird eine gasförmige Fraktion erhalten, die über 98 Massen-% MTBE enthält. Der Gehalt an 2-Methoxybutan im Destillat wurde auf 2000 Massen-ppm eingestellt (siehe Tabelle 12). Das Aceton gelangt in dieser Kolonne komplett ins Destillat.

**Tabelle 12: Zusammensetzung des Sumpfsprodukts (V) und des Destillats (VI) der Kolonne K2 sowie des Reaktoraustrags (VII) aus dem Reaktor R für Beispiel 4**

| | Sumpfprod. K2 (V) | Destillat K2 (VI) | Reaktoraustrag (VII) |
|---|---|---|---|
| Massenstrom [kg/h] | 20,9 | 1024,3 | 1024,3 |
| Massenanteile [kg/kg] | | | |
| Dimethylether | | | 0,002300 |
| Isobutan | | | |
| Isobuten | | | 0,539935 |
| n-Butan | | | |
| Butadien | | | |
| 1-/2-Butene | | | 0,000205 |
| C5-Kohlenwasserstoffe | | 0,000001 | 0,000001 |
| MTBE | 0,826570 | 0,985827 | 0,147638 |
| 2-Methoxybutan | 0,078959 | 0,002000 | 0,001678 |
| Methanol | | | 0,301598 |
| Tert.-Butanol | | 0,012146 | 0,003024 |
| Wasser | | | 0,003116 |
| Diisobuten | 0,094471 | | 0,000480 |
| Aceton | | 0,000026 | 0,000026 |

Die MTBE-Fraktion (VI) wird nach weiterer Aufheizung auf Reaktionstemperatur, gemäß Figur 5 dem Spaltungsreaktor in Verfahrensschritt d) zugeführt. Der Spaltungsreaktor wird mit einem Reaktorvolumen von 0,9 m3 modelliert, wobei eine Füllung mit einem Katalysator angenommen wird, der formal aus Magnesiumoxid, Aluminiumoxid und Siliziumoxid besteht und dessen Herstellung im Patent DE 102006040432.7 beschrieben ist.

Der Reaktor wird bei 289°C und 0,7 MPa_{(abs)} gefahren. Bei diesen Reaktionsbedingungen ergibt sich ein MTBE-Umsatz von ca. 85 %, der Umsatz von 2-Methoxybutan beträgt ca. 16 %. Aufgrund der Begrenzung des Anteils an 2-Methoxybutan auf 2000 Massen-ppm im Reaktorzulauf ist aber trotz der Spaltung von 2-Methoxybutan zu 1- und 2-Buten eine marktübliche Spezifikation für lineare Butene im Isobutenprodukt nicht gefährdet. Für Aceton wird, gemäß Beispiel 1, keine Umsetzung angenommen. Die Zusammensetzung des Reaktoraustrags (IV) zeigt Tabelle 12.

Der Reaktoraustrag (VII) wird teilweise kondensiert und zweiphasig der Kolonne K3 zugeführt. Die Kolonne hat 45 theoretische Stufen und wird bei einem Rücklaufverhältnis von 0,3 und bei einem Druck von 0,65 MPa(abs) betrieben. Die Zugabe des Zulaufstromes (VI) erfolgt oberhalb Stufe 28, gezählt von oben. Das Sumpfprodukt besteht überwiegend aus nicht umgesetztem MTBE (ca. 33 Massen-%) und Methanol (ca. 64 Massen-%) sowie dem überwiegenden Teil der im Zulaufstrom enthaltenen Acetons (56 ppm), siehe Tabelle 13. Strom (VIII) wird in die MTBE-Synthese S1 zurückgeführt.

Das Kopfprodukt (IX) ist Isobuten mit einer Reinheit von größer 95 Massen-% Isobuten. Die in einer typischen Isobutenspezifikation geforderten Grenzen für lineare Butene (< 1000 Massen-ppm) und C5-Kohlenwasserstoffe (< 1000 Massen-ppm) werden sicher eingehalten, siehe auch Tabelle 1. Aceton ist nur noch in einer Konzentration von 3 ppm enthalten, was aber noch ca. 6 Massen-% der im Zulaufstrom (VII) enthaltenen Menge an Aceton entspricht.

**Tabelle 13: Zusammensetzung des Sumpfsprodukts (VIII) und des Destillats (IX) der Kolonne K3 für Beispiel 4**

| | Sumpfprod. K3 (VIII) | Destillat K2 (IX) |
|---|---|---|
| Massenstrom [kg/h] | 446,4 | 577,9 |
| Massenanteile [kg/kg] | | |
| Dimethylether | 0,000019 | 0,004061 |
| Isobutan | | |
| Isobuten | 0,000500 | 0,956601 |
| n-Butan | | |
| Butadien | | |
| 1-/2-Butene | 0,000025 | 0,000344 |
| C5-Kohlenwasserstoffe | 0,000001 | |
| MTBE | 0,338774 | 0,000001 |
| 2-Methoxybutan | 0,003850 | |
| Methanol | 0,642355 | 0,038392 |
| Tert.-Butanol | 0,006940 | |
| Wasser | 0,006377 | 0,000598 |
| Diisobuten | 0,001102 | |
| Aceton | 0,000056 | 0,000003 |

In der Extraktion K4 wird der Strom (IX) mit Wasser (XI) gewaschen, um das noch enthaltene Methanol zu entfernen. Die Kolonne K4 hat 5 theoretische Stufen und wird bei einbem Druck von 1,2 MPa_{(abs)} betrieben. Die Aufgabe des Frischwassers (XIII) erfolgt auf Stufe 1, gezählt von oben, der Abzug des methanolbeladenen Wassers (XI) (wässrige Phase) erfolgt auf Stufe 5 und der Abzug feuchten, methanolfreien Isobutenstroms (X) (organische Phase) auf Stufe 1.

**Tabelle 14: Zusammensetzung des Frischwassers, der organischen Phase (X) und der wässrigen Phase (XI) der Kolonne K4 für Beispiel 4**

| | Frischwasser (XIII) | organische Phase K4 (X) | wässrige Phase K4 (XI) |
|---|---|---|---|
| Massenstrom [kg/h] | 100,0 | 555,5 | 122,4 |
| Massenanteile [kg/kg] | | | |
| Dimethylether | | 0,003929 | 0,001342 |
| Isobutan | | | |
| Isobuten | | 0,994815 | 0,001612 |
| n-Butan | | | |
| Butadien | | | |
| 1-/2-Butene | | 0,000358 | 0,000001 |
| C5-Kohlenwasserstoffe | | | |
| MTBE | | 0,000001 | |
| 2-Methoxybutan | | | |
| Methanol | | 0,000002 | 0,181264 |
| Tert.-Butanol | | | |
| Wasser | 1,000000 | 0,000892 | 0,815776 |
| Diisobuten | | | |
| Aceton | | 0,000002 | 0,000005 |

Die Zusammensetzungen des Frischwassers, der organischen Phase (X) und der wässrigen Phase (XI) der Kolonne K4 sind in Tabelle 14 dargestellt.

Optional kann Strom (XI) in einer weiteren Destillationskolonne K5, wie in Figur 6 dargestellt, wieder in Wasser und Methanol aufgetrennt werden.

Der feuchte Isobutenstrom (X) ist nahezu methanolfrei, enthält aber noch DME. Das Aceton wurde geringfügig in der Extraktion abgereichert.

Der Isobutenstrom (X) wird der Kolonne K6 zugeführt, um Restwasser und Dimethylether abzutrennen. Die Kolonne hat 70 theoretische Stufen und wird bei einem Rücklaufverhältnis von 81 und bei einem Druck von 1,0 MPa_{(abs)} betrieben. Die Zugabe des Zulaufstromes (X) erfolgt oberhalb Stufe 8, gezählt von oben. Die Kolonne hat einen Kopfdekanter, an dem Wasser (XV) als zweite Phase abgezogen wird.

Die Zusammensetzungen des Destillats (XIV), der wässrigen Phase (XV) des Kopfdekanters und des Sumpfprodukts (XVI) der Kolonne K6 zeigt Tabelle 15.

**Tabelle 15: Zusammensetzung des Destillats (XIV), der wässrigen Phase (XV) des Kopfdekanters und des Sumpfprodukts (XVI) der Kolonne K6 für Beispiel 4**

| | Destillat K6 (XIV) | wäss. Phase Dekanter K6 (XV) | Sumpfprod. K6 (XVI) |
|---|---|---|---|
| Massenstrom [kg/h] | 18,2 | 0,3 | 537,0 |
| Massenanteile [kg/kg] | | | |
| Dimethylether | 0,119747 | 0,014149 | |
| Isobutan | | | |
| Isobuten | 0,870535 | 0,000425 | 0,999629 |
| n-Butan | | | |
| Butadien | | | |
| 1-/2-Butene | 0,000067 | | 0,000368 |
| C5-Kohlenwasserstoffe | | | |
| MTBE | | | 0,000001 |
| 2-Methoxybutan | | | |
| Methanol | 0,000061 | 0,000665 | |
| Tert.-Butanol | | | |
| Wasser | 0,009590 | 0,984760 | |
| Diisobuten | | | |
| Aceton | | | 0,000002 |

Das Destilat (XIV) der Kolonne K6 enthält noch ca. 87 Massen-% Isobuten. DME kann optional in einer weiteren Kolonne höher aufkonzentriert und das Isobuten in die Kolonne K6 zurück geführt werden.

Das Sumpfprodukt ist Isobuten mit einer Reinheit größer 99,9 Massen-% und genügt der Spzifikation gemäß Tabelle 1. Insbesondere sind die Summe der Oxygenates kleiner 10 ppm, die Acetonkonzentration liegt bei 2 ppm.

### Beispiel 5: (nicht erfindungsgemäß)

Das Beispiel 4 dient als Vergleichsbeispiel und beinhaltet nicht das erfindungsgemäße Verfahren. Demnach wird ein Verfahren ähnlich Figur 5 betrachtet, wobei allerdings Verfahrschritt b), d.h. die Ausschleusung des Acetons im Seitenabzug der Kolonne K1, entfällt. An Stelle der der Kolonne K1 mit Seitenabzug wird eine einfache Destillationskolonne K1* gemäß Beispiel 2a gesetzt. Alle anderen Verfahrensschritte und Kolonnenkonfigurationen bleiben gegenüber Beispiel 5 unverändert.

Als Zulauf zur MTBE-Synthese S1 wird analog zu Beispiel 4 ein C4-Kohlenwasserstoffstrom (XX) von 1800 kg/h mit unveränderter Zusammensetzung angenommen, siehe Tabelle 10. In Tabelle 16 ist die Zusammensetzung und der Massenstrom des zugeführten Frischmethanols (XX) aufgeführt. Zusammensetzung und Massenstrom des aus Verfahrenschritt e) kommenden des Rückführstroms (VIII) (Sumpfprodukt Kolonne K3) sind in Tabelle 19 dargestellt. Die Frischmethanolmenge wurde so eingestellt, dass sich wieder ein molares Verhältnis von Methanol zu Isobuten im Zulauf zur MTBE-Synthese von 1,13 ergibt.

**Tabelle 16: Zusammensetzung des Frischmethanols (XXI) sowie des Destillats (XII) und des Sumpfprodukts (I) (Einsatz-MTBE) der Kolonne KS1-1 für Beispiel 5**

| | Destillat KS1-1 (XXII) | Methanol (XXI) | Einsatz-MTBE (I) |
|---|---|---|---|
| Massenstrom [kg/h] | | 90,8 | 1129,3 |
| Massenanteile [kg/kg] | | | |
| Dimethylether | | | |
| Isobutan | | | |
| Isobuten | | | |
| n-Butan | | | |
| Butadien | | | |
| 1-/2-Butene | | | 0,000453 |
| C5-Kohlenwasserstoffe | | | 0,000824 |
| MTBE | | | 0,969930 |
| 2-Methoxybutan | | | 0,003418 |
| Methanol | | 1,000000 | 0,009500 |
| Tert.-Butanol | | | 0,012502 |
| Wasser | | | 0,000004 |
| Diisobuten | | | 0,001776 |
| Aceton | | | 0,001594 |

Der C4-Kohlenwasserstoffstrom (XX), das Frischmethanol (XXI) und der methanolhaltige Rückführungsstrom (VIII) werden in Verfahrensschritt S1 vermischt und einer MTBE-Synthese zugeführt. Die Anordnung der Reaktoren, Reaktorgrößen sowie Eintrittstemperaturen bleiben gegenüber Beispiel 4 unverändert. Unter diesen Bedingungen ergibt sich wieder ein Gesamtumsatz von Isobuten von ca. 96 %. Der Reaktoraustrag aus dem Reaktor RS1-3 wird wieder der Kolonne KS1-1 zugeführt. Stufenzahl, Rücklaufverhältnis und Kolonnendruck bleiben gegenüber Beispiel 4 unverändert. Zusammensetzung und Massenstrom von Destillat (XXII) und Sumpfprodukt (I) (Einsatz-MTBE) sind in Tabelle 16 dargestellt. Zu beachten ist, dass aufgrund fehlender Abtrennung in Kolonne K1 und der damit verbundenen erhöhten Acetonkonzentration im Rücklaufstrom (VIII) die Acetonkonzentration im Einsatz-MTBE (I) gegenüber Beispiel 4 deutlich auf ca. 1600 ppm angestiegen ist.

Aus dem MTBE-Strom (I) werden in der Kolonne K1* die C4- und C5-Kohlenwasserstoffe abgetrennt. Die Kolonne K1* hat, wie in Bespiel 2a, 40 theoretische Stufen und wird bei einem Druck von 0,35 MPa_{(abs)} betrieben. Die Aufgabe des Zulaufs (I) erfolgt oberhalb Stufe 15, gezählt von oben. Um analog zu Beispiel 2a eine Konzentration von 50 ppm C4- und C5-Kohlenwasserstoffe im Sumpfprodukt (IV*) zu erreichen, wird die Kolonne bei einem Rücklauf von 826 kg/h betrieben. Tabelle 17 zeigt die Zusammensetzung des Destillatstroms (II*) und des Sumpfstroms (IV*I) der Kolonne K1*.

**Tabelle 17: Zusammensetzung des Destillats (II*) und des Sumpfsprodukts (IV*) für Kolonne K1* für Beispiel 5**

| | Destillat K1 (II*) | Sumpfprod. K1* (IV*) |
|---|---|---|
| Massenstrom [kg/h] | 1,9 | 1127,4 |
| Massenanteile [kg/kg] | | |
| Dimethylether | | |
| Isobutan | | |
| Isobuten | | |
| n-Butan | 0,000004 | |
| Butadien | | |
| 1-/2-Butene | 0,273941 | |
| C5-Kohlenwasserstoffe | 0,467714 | 0,000050 |
| MTBE | 0,150000 | 0,971288 |
| 2-Methoxybutan | 0,000086 | 0,003424 |
| Methanol | 0,107314 | 0,009338 |
| Tert.-Butanol | | 0,012523 |
| Wasser | 0,000006 | 0,000004 |
| Diisobuten | | 0,001778 |
| Aceton | 0,000936 | 0,001595 |

Aceton wird in dieser Fahrweise, wie schon in Beispiel 2a gezeigt, nicht abgetrennt und aus dem Verfahren ausgeschleust.

Die MTBE-Strom (IV*) wird der Kolonne K2 zugeführt, in der Diisobuten und 2-Methoxybutan über Sumpf (V) abgetrennt werden. Stufenzahl und Kolonnendruck bleiben gegenüber Beispiel 4 unverändert. Das Rücklaufverhältnis beträgt 2,9. Zusammensetzungen und Massenströme von Kopf- und Sumpfprdoukt der Kolonne K2 sind in Tabelle 18 dargestellt. Das Aceton gelangt in dieser Kolonne wieder komplett ins Destillat.

**Tabelle 18: Zusammensetzung des Sumpfsprodukts (V) und des Destillats (VI) der Kolonne K2 sowie des Reaktoraustrags (VII) aus dem Reaktor R für Beispiel 5**

| | Sumpfprod. K2 (V) | Destillat K2 (VI) | Reaktoraustrag (VII) |
|---|---|---|---|
| Massenstrom [kg/h] | 22,5 | 1104,9 | 1104,9 |
| Massenanteile [kg/kg] | | | |
| Dimethylether | | | 0,002344 |
| Isobutan | | | |
| Isobuten | | | 0,533905 |
| n-Butan | | | |
| Butadien | | | |
| 1-/2-Butene | | | 0,000205 |
| C5-Kohlenwasserstoffe | | 0,000051 | 0,000051 |
| MTBE | 0,837882 | 0,974011 | 0,145868 |
| 2-Methoxybutan | 0,073196 | 0,002000 | 0,001678 |
| Methanol | | 0,009529 | 0,307412 |
| Tert.-Butanol | | 0,012778 | 0,003182 |
| Wasser | | 0,000004 | 0,003253 |
| Diisobuten | 0,088922 | | 0,000474 |
| Aceton | | 0,001628 | 0,001628 |

Die MTBE-Fraktion (VI) wird, nach Verdampfung und weiterer Aufheizung auf Reaktionstemperatur, gemäß Figur 5 dem Spaltungsreaktor in Verfahrensschritt d) zugeführt. Reaktorvolumen, Katalysatorfüllung und Reaktionsdruck bleiben gegenüber Beispiel 4 unverändert. Die Reaktortemperatur wird auf 292°C angehoben, um wieder einen MTBE-Umsatz von ca. 85 % und einen 2-Methoxybutan-Umsatz von ca. 16 % zu erreichen. Für Aceton wird wieder keine Umsetzung angenommen. Die Zusammensetzung des Reaktoraustrags (IV) zeigt Tabelle 18.

Der Reaktoraustrag (VII) wird teilweise kondensiert und zweiphasig der Kolonne K3 zugeführt. Stufenzahl, Rücklaufverhältnis und Kolonnendruck bleiben gegenüber Beispiel 4 unverändert. Wie in Beispiel 4 werden wieder nur ca. 6 Massen-% des im Zulaufstroms (VII) enthaltenen Acetons ins Kopfprodukt (IX) destilliert, ca. 94 Massen-% des Acetons gelangen ins Sumpfprodukt (VIII), siehe Tabelle 19. Da jedoch die Konzentration im Zulauf aufgrund einer fehlenden Aceton-Ausschleusung sehr hoch ist, gelangen ca. 170 ppm Aceton ins Kopfprodukt.

**Tabelle 19: Zusammensetzung des Sumpfsprodukts (VIII) und des Destillats (IX) der Kolonne K3 für Beispiel 5**

| | Sumpfprod. K3 (XIII) | Destillat K2 (IX) |
|---|---|---|
| Massenstrom [kg/h] | 488,4 | 616,5 |
| Massenanteile [kg/kg] | | |
| Dimethylether | 0,000021 | 0,004184 |
| Isobutan | | |
| Isobuten | 0,000500 | 0,956450 |
| n-Butan | | |
| Butadien | | |
| 1-/2-Butene | 0,000025 | 0,000348 |
| C5-Kohlenwasserstoffe | 0,000082 | 0,000026 |
| MTBE | 0,330005 | 0,000001 |
| 2-Methoxybutan | 0,003796 | |
| Methanol | 0,647238 | 0,038214 |
| Tert.-Butanol | 0,007198 | |
| Wasser | 0,006596 | 0,000604 |
| Diisobuten | 0,001073 | |
| Aceton | 0,003466 | 0,000172 |

Strom (VIII) wird in die MTBE-Synthese S1 zurückgeführt, das Kopfprodukt (IX) wird analog zu Beispiel 4 in den Kolonnen K4 und K6 weiter aufgearbeitet. Stufenzahl und Kolonnendruck bleiben für beide Kolonnen gegenüber Beispiel 4 unverändert. Die Kolonne K6 wird bei einem Rücklaufverhältnis von 73 betrieben. Die entsprechenden Zusammensetzungen der Ströme zeigen Tabelle 20 und 21.

**Tabelle 20: Zusammensetzung des Frischwassers, der organischen Phase (X) und der wässrigen Phase (XI) der Kolonne K4 für Beispiel 5**

| | Frischwasser (XIII) | Organische Phase K4 (X) | Wässrige Phase K4 (XI) |
|---|---|---|---|
| Massenstrom [kg/h] | 100,0 | 592,7 | 123,8 |
| Massenanteile [kg/kg] | | | |
| Dimethylether | | 0,004060 | 0,001401 |
| Isobutan | | | |
| Isobuten | | 0,994541 | 0,001717 |
| n-Butan | | | |
| Butadien | | | |
| 1-/2-Butene | | 0,000361 | 0,000001 |
| C5-Kohlenwasserstoffe | | 0,000027 | |
| MTBE | | 0,000001 | |
| 2-Methoxybutan | | | |
| Methanol | | 0,000003 | 0,190243 |
| Tert.-Butanol | | | |
| Wasser | 1,000000 | 0,000894 | 0,806316 |
| Diisobuten | | | |
| Aceton | | 0,000112 | 0,000323 |

**Tabelle 21: Zusammensetzung des Destillats (XIV), der wässrigen Phase (XV) des Kopfdekanters und des Sumpfprodukts (XVI) der Kolonne K6 für Beispiel 5**

| | Destillat K6 (XIV) | wäss. Phase Dekanter K6 (XV) | Sumpfprod. K6 (XVI) |
|---|---|---|---|
| Massenstrom [kg/h] | 20,1 | 0,3 | 572,3 |
| Massenanteile [kg/kg] | | | |
| Dimethylether | 0,119758 | 0,014157 | |
| Isobutan | | | |
| Isobuten | 0,870501 | 0,000426 | 0,999483 |
| n-Butan | | | |
| Butadien | | | |
| 1-/2-Butene | 0,000069 | | 0,000372 |
| C5-Kohlenwasserstoffe | | | 0,000028 |
| MTBE | | | 0,000001 |
| 2-Methoxybutan | | | |
| Methanol | 0,000081 | 0,000875 | |
| Tert.-Butanol | | | |
| Wasser | 0,009589 | 0,984537 | |
| Diisobuten | | | |
| Aceton | 0,000003 | 0,000005 | 0,000116 |

Wie aus Tabelle 20 und 21 zu erkennen, wird zwar Aceton in der Extraktionskolonne K4 noch leicht abgereichert, dennoch wird die Isobutenprodukt (XVI) mit 116 ppm ein Wert erreicht, der die geforderte Spezifikation in Tabelle 1 (Oxygenates kleiner 10 ppm) deutlich überschreitet.

Durch die Gegenüberstellung von Beispiel 4 (erfindungsgemäß) und Beispiel 5 (nicht erfindungsgemäß) konnten sehr anschaulich die Vorteile des erfindungsgemäßen Verfahrens aufgezeigt werden. Durch den fehlenden Verfahrensschritt b) (Acetonausschleusung) reichert sich Aceton im Kreislauf an und gelangt in unerwünscht hohen Konzentrationen ins Isobutenprodukt.

### Bezugszeichenliste:

- (I), (Ia): MTBE-haltiger Strom bzw. technisches MTBE
- (II): C4- und C5- Kohlenwasserstoffe enthaltendes Kopfprodukt von K1
- (III): Aceton, Methanol und MTBE enthaltender Seitenstrom von K1
- (IV): MTBE enthaltendes Sumpfprodukt von K1
- (V): Sumpfprodukt von K2 enthaltend höher als MTBE siedende Komponenten
- (VI): MTBE-haltiges Kopfprodukt von K2
- (VII): Spaltprodukt von K2 enthaltend zumindest MTBE, Isobuten und Methanol
- (VIII): MTBE und Methanol enthaltendes Sumpfprodukt von K3
- (IX): Isobuten enthaltendes Kopfprodukt von K3
- (X): Isobuten angereicherter Strom von K4
- (XI): Methanol enthaltender Extraktionsstrom von K4
- (XII): Methanol enthaltendes Kopfprodukt von K5
- (XIII): Sumpfprodukt von K5 und/oder Extraktionsmittel für K4
- (XIV): Wasser enthaltendes Kopfprodukt von K6
- (XV): Dimethylether enthaltender Seitenstrom von K6
- (XVI): Isobuten enthaltendes Sumpfprodukt von K6
- (XVII): MTBE enthaltendes Kopfprodukt von K7
- (XVIII): Methanol enthaltendes Sumpfprodukt von K7
- (XX): isobutenhaltiger Strom für S1
- (XXI): methanolhaltiger Strom für S1
- (XXII): MTBE-haltiges Produkt von S1
- (K1): Destillationskolonne in Verfahrensschritt b)
- (K2): Destillationskolonne in Verfahrensschritt c)
- (K3): Destillationskolonne in Verfahrensschritt e)
- (K4): Destillationskolonne in Verfahrensschritt f)
- (K5): Destillationskolonne
- (K6): Destillationskolonne
- (K7): Destillationskolonne
- (R): MTBE-Spaltung in Verfahrensschritt d)
- (S1): MTBE-Synthese

## Patentansprüche

1. Verfahren zur Aufreinigung von technischem Methyl-tert.-butylether (MTBE), das folgende Schritte umfasst:
a) Bereitstellen von technischem MTBE (I), enthaltend mindestens MTBE, Methanol, C4-Kohlenwasserstoffe, C5-Kohlenwasserstoffe und Aceton; und
b) destillatives Trennen des technischen MTBE (I) in ein C4- und C5-Kohlenwasserstoffe enthaltendes Kopfprodukt (II), einen Aceton, Methanol und MTBE enthaltenden Seitenstrom (III), und ein MTBE enthaltendes Sumpfprodukt (IV).

2. Verfahren nach Anspruch 1, **dadurch gekennzeichnet, dass** das destillative Trennen in Verfahrensschritt b) so durchgeführt wird, dass das Sumpfprodukt (IV) weniger als 50 Massen-% des im technischen MTBE (I) enthaltenen Acetons enthält.

3. Verfahren nach Anspruch 1 oder 2, **dadurch gekennzeichnet, dass** das destillative Trennen in Verfahrensschritt b) in einer Destillationskolonne durchgeführt wird und der Seitenstrom (III) flüssig entnommen wird.

4. Verfahren nach zumindest einem der Ansprüche 1 bis 3, **dadurch gekennzeichnet, dass** das MTBE enthaltende Sumpfprodukt (IV) aus Verfahrensschritt b) in einem Verfahrensschritt c) in einer weiteren Destillation in ein MTBE-haltiges Kopfprodukt (VI) und ein Sumpfprodukt (V), das höher als MTBE siedende Komponenten aufweist, aufgetrennt wird.

5. Verfahren nach Anspruch 4, **dadurch gekennzeichnet, dass** das destillative Trennen in Verfahrensschritt c) so durchgeführt wird, dass das MTBE-haltige Kopfprodukt (VI) eine Konzentration von weniger als 2500 Massen-ppm an 2-Methoxybutan aufweist.

6. Verfahren nach einem der Ansprüche 1 bis 5, **dadurch gekennzeichnet, dass** das MTBE enthaltende Sumpfprodukt (IV) oder das MTBE-haltige Kopfprodukt (VI) weniger als 50 Massen-ppm an Aceton enthält.

7. Verfahren nach einem der Ansprüche 1 bis 6, **dadurch gekennzeichnet, dass** das MTBE enthaltende Sumpfprodukt (IV) oder das MTBE-haltige Kopfprodukt (VI) in einem Methyl-tert.-butylether (MTBE) Verfahrensschritt d) katalytisch gespalten wird unter Erhalt eines Spaltproduktes (VII), welches zumindest MTBE, Isobuten und Methanol enthält.

8. Verfahren nach Anspruch 7, **dadurch gekennzeichnet, dass** die Spaltung an einem festen Katalysator in der Gasphase in einem Temperaturbereich von 150 bis 500 °C durchgeführt wird.

9. Verfahren nach Anspruch 7 oder 8, **dadurch gekennzeichnet, dass** das in Verfahrensschritt d) gewonnene Spaltprodukt (VII) in einem Verfahrensschritt e) in einer weiteren Destillation in ein Isobuten enthaltendes Kopfprodukt (IX) und ein MTBE und Methanol enthaltendes Sumpfprodukt (VIII) aufgetrennt wird.

10. Verfahren nach Anspruch 9, **dadurch gekennzeichnet, dass** das in Verfahrensschritt e) gewonnene und Isobuten enthaltende Kopfprodukt (IX) weniger als 10 Massen-ppm an Aceton enthält.

11. Verfahren nach Anspruch 9 oder 10, **dadurch gekennzeichnet, dass** aus dem in Verfahrensschritt e) gewonnenen und Isobuten enthaltenden Kopfprodukt (IX) in einem weiteren Verfahrensschritt f) Methanol durch Extraktion und/oder Dimethylether durch Destillation entfernt wird.

12. Verfahren nach Anspruch 11, **dadurch gekennzeichnet, dass** in dem Verfahrensschritt f) aus dem Isobuten enthaltenden Kopfprodukt (IX) das Methanol durch Extraktion mittels eines Extraktionsmittels (XIII) und Abführen eines Methanol enthaltenden Extraktionsstroms (XI) und Abführen eines Isobuten angereicherten Stroms (X) entfernt wird.

13. Verfahren nach Anspruch 12, **dadurch gekennzeichnet, dass** der Methanol enthaltende Extraktionsstrom (XI) in einem weiteren Verfahrensschritt in einer Destillation in ein Methanol enthaltendes Kopfprodukt (XII) und ein das Extraktionsmittel (XIII) enthaltendes Sumpfprodukt (XIII) aufgetrennt wird.

14. Verfahren nach einem der Ansprüche 9 bis 13, **dadurch gekennzeichnet, dass** das in Verfahrensschritt e) gewonnene und MTBE und Methanol enthaltende Sumpfprodukt (VIII) ganz oder teilweise in Verfahrensschritt b) zurückgeführt wird.

15. Verfahren nach Anspruch 14, **dadurch gekennzeichnet, dass** der MTBE und Methanol enthaltende Sumpfstrom (VIII) in einem weiteren Verfahrensschritt in einer Destillation in ein Methanol enthaltendes Sumpfprodukt (XVIII) und ein MTBE enthaltendes Kopfprodukt (XVII) aufgetrennt wird, wobei das Kopfprodukt (XVII) ganz oder teilweise in Verfahrensschritt b) zurückgeführt wird.

16. Verfahren nach Anspruch 14, **dadurch gekennzeichnet, dass** der MTBE und Methanol enthaltende Sumpfstrom (VIII) sowie mindestens ein weiterer methanolhaltiger Strom (XXI) und ein isobutenhaltiger Strom (XX) einer MTBE-Synthese (S1) zugeführt werden und ein MTBE-haltiges Produkt (XXII) ganz oder teilweise in Verfahrensschritt b) zurückgeführt wird.

## Claims

1. Process for purifying technical methyl-tert-butylether (MTBE), which comprises the following steps:
a) providing technical MTBE (I) comprising at least MTBE, methanol, C4 hydrocarbons, C5 hydrocarbons and acetone; and
b) distillatively separating the technical MTBE (I) into a top product (II) comprising C4 and C5 hydrocarbons, a side stream (III) comprising acetone, methanol and MTBE, and a bottom product (IV) comprising MTBE.

2. Process according to Claim 1, **characterized in that** the distillative separation in process step b) is performed in such a way that the bottom product (IV) comprises less than 50% by mass of the acetone present in the technical MTBE (I).

3. Process according to Claim 1 or 2, **characterized in that** the distillative separation in process step b) is performed in a distillation column and the side stream (III) is withdrawn in liquid form.

4. Process according to at least one of Claims 1 to 3, **characterized in that** the MTBE-comprising bottom product (IV) from process step b) is separated in a process step c) in a further distillation into an MTBE-containing top product (VI) and a bottom product (V) comprising higher-boiling components than MTBE.

5. Process according to Claim 4, **characterized in that** the distillative separation in process step c) is performed in such a way that the MTBE-containing top product (VI) has a concentration of less than 2500 ppm by mass of 2-methoxybutane.

6. Process according to any of Claims 1 to 5, **characterized in that** the MTBE-comprising bottom product (IV) or the MTBE-containing top product (VI) comprises less than 50 ppm by mass of acetone.

7. Process according to any of Claims 1 to 6, **characterized in that** the MTBE-comprising bottom product (IV) or the MTBE-containing top product (VI) is catalytically cracked in a process step d) to obtain a cracking product (VII) comprising at least MTBE, isobutene and methanol.

8. Process according to Claim 7, **characterized in that** the cracking is performed over a solid catalyst in the gas phase within a temperature range from 150 to 500°C.

9. Process according to Claim 7 or 8, **characterized in that** the cracking product (VII) obtained in process step d) is separated in a process step e) in a further distillation into an isobutene-comprising top product (IX) and an MTBE- and methanol-comprising bottom product (VIII).

10. Process according to Claim 9, **characterized in that** the top product (IX) which is obtained in process step e) and comprises isobutene comprises less than 10 ppm by mass of acetone.

11. Process according to Claim 9 or 10, **characterized in that** methanol is removed by extraction and/or dimethyl ether by distillation in a further process step f) from the top product (IX) which is obtained in process step e) and comprises isobutene.

12. Process according to Claim 11, **characterized in that** the methanol is removed in process step f) from the isobutene-comprising top product (IX) by extraction by means of an extractant (XIII) and removing a methanol-comprising extraction stream (XI) and removing an isobutene-enriched stream (X).

13. Process according to Claim 12, **characterized in that** the methanol-comprising extraction stream (XI) is separated in a further process step in a distillation into a methanol-comprising top product (XII) and a bottom product (XIII) comprising the extractant (XIII).

14. Process according to any of Claims 9 to 13, **characterized in that** the bottom product (VIII) which is obtained in process step e) and comprises MTBE and methanol is fully or partly recycled into process step b) .

15. Process according to Claim 14, **characterized in that** the MTBE- and methanol-comprising bottom stream (VIII) is separated in a further process step in a distillation into a methanol-comprising bottom product (XVIII) and an MTBE-comprising top product (XVII), said top product (XVII) being recycled fully or partly into process step b) .

16. Process according to Claim 14, **characterized in that** the MTBE- and methanol-comprising bottom stream (VIII) and at least one further methanol-containing stream (XXI) and an isobutene-containing stream (XX) are supplied to an MTBE synthesis (S1) and an MTBE-containing product (XXII) is recycled fully or partly into process step b).

## Revendications

1. Procédé pour la purification de méthyl-tert-butyléther (MTBE) de qualité technique, qui présente les étapes suivantes :
a) mise à disposition de MTBE (I) de qualité technique, contenant au moins du MTBE, du méthanol, des hydrocarbures en C₄, des hydrocarbures en C₅ et de l'acétone ; et
b) séparation par distillation du MTBE (I) de qualité technique en un produit de tête (II) contenant des hydrocarbures en C₄ et en C₅, en un flux latéral (III) contenant de l'acétone, du méthanol et du MTBE et en un produit de fond (IV) contenant du MTBE.

2. Procédé selon la revendication 1, **caractérisé en ce que** la séparation par distillation dans l'étape de procédé b) est réalisée de manière telle que le produit de fond (IV) contient moins de 50% en masse de l'acétone contenue dans le MTBE (I) de qualité technique.

3. Procédé selon la revendication 1 ou 2, **caractérisé en ce que** la séparation par distillation dans l'étape de procédé b) est réalisée dans une colonne de distillation et le flux latéral (III) est prélevé sous forme liquide.

4. Procédé selon au moins l'une quelconque des revendications 1 à 3, **caractérisé en ce que** le produit de fond (IV) contenant du MTBE de l'étape de procédé b) est séparé dans une étape de procédé c), dans une autre distillation, en un produit de tête (VI) contenant du MTBE et en un produit de fond (V) qui présente les constituants à point d'ébullition supérieur à celui du MTBE.

5. Procédé selon la revendication 4, **caractérisé en ce que** la séparation par distillation dans l'étape de procédé c) est réalisée de manière telle que le produit de tête (VI) contenant du MTBE présente une concentration en 2-méthoxybutane inférieure à 2500 ppm en masse.

6. Procédé selon l'une quelconque des revendications 1 à 5, **caractérisé en ce que** le produit de fond (IV) contenant du MTBE ou le produit de tête (VI) contenant du MTBE contient moins de 50 ppm en masse d'acétone.

7. Procédé selon l'une quelconque des revendications 1 à 6, **caractérisé en ce que** le produit de fond (IV) contenant du MTBE ou le produit de tête (VI) contenant du MTBE est dissocié catalytiquement dans une étape de procédé d) avec obtention d'un produit dissocié (VII) qui contient au moins du MTBE, de l'isobutène et du méthanol.

8. Procédé selon la revendication 7, **caractérisé en ce que** la dissociation est réalisée sur un catalyseur solide en phase gazeuse dans une plage de température de 150 à 500°C.

9. Procédé selon la revendication 7 ou 8, **caractérisé en ce que** le produit dissocié (VII) obtenu dans l'étape de procédé d) est séparé dans une étape de procédé e) dans une autre distillation en un produit de tête (IX) contenant de l'isobutène et un produit de fond (VIII) contenant du MTBE et du méthanol.

10. Procédé selon la revendication 9, **caractérisé en ce que** le produit de tête (IX) obtenu dans l'étape de procédé e) et contenant de l'isobutène contient moins de 10 ppm en masse d'acétone.

11. Procédé selon la revendication 9 ou 10, **caractérisé en ce qu'**on élimine, du produit de tête (IX) obtenu dans l'étape de procédé e) et contenant de l'isobutène, dans une autre étape de procédé f), le méthanol par extraction et/ou le diméthyléther par distillation.

12. Procédé selon la revendication 11, **caractérisé en ce que** dans l'étape de procédé f), on élimine, du produit de tête (IX) contenant de l'isobutène, le méthanol par extraction au moyen d'un agent extraction (XIII) et par évacuation d'un flux d'extraction (XI) contenant du méthanol et par évacuation d'un flux (X) enrichi en isobutène.

13. Procédé selon la revendication 12, **caractérisé en ce que** le flux d'extraction (XI) contenant du méthanol est séparé dans une autre étape de procédé, dans une distillation, en un produit de tête (XII) contenant du méthanol et en un produit de fond (XIII) contenant l'agent d'extraction (XIII).

14. Procédé selon l'une quelconque des revendications 9 à 13, **caractérisé en ce que** le produit de fond (VIII) obtenu dans l'étape de procédé e) et contenant du MTBE et du méthanol est recyclé totalement ou partiellement dans l'étape de procédé b).

15. Procédé selon la revendication 14, **caractérisé en ce que** le produit de fond (VIII) contenant du MTBE et du méthanol est séparé dans une autre étape de procédé, dans une distillation, en un produit de fond (XVIII) contenant du méthanol et en un produit de tête (XVII) contenant du MTBE, le produit de tête (XVII) étant recyclé totalement ou partiellement dans l'étape de procédé b).

16. Procédé selon la revendication 14, **caractérisé en ce que** le produit de fond (VIII) contenant du MTBE et du méthanol ainsi qu'au moins un autre flux (XXI) contenant du méthanol et un flux (XX) contenant de l'isobutène sont introduits dans une synthèse de MTBE (SI) et un produit (XXII) contenant du MTBE est recyclé totalement ou partiellement dans l'étape de procédé b).
